# EUROPEAN PATENT APPLICATION

(11) **EP 1 541 582 A1**
(43) Date of publication of application: **15.06.2005**
(21) Application number: 03792815.7
(22) Date of filing: 22.08.2003
(51) Int. Cl.: C07H 19/23, C07D 207/456, C07D 209/32, C07D 403/14, C07D 487/14, B01J 27/053, B01J 27/128, B01J 27/25, B01J 31/04, B01J 31/22, B01J 31/28

(54) **PROCESS FOR PRODUCING INDOLOPYRROLOCARBAZOLE DERIVATIVE**

(30) Priority: 23.08.2002 JP 2002244173
(71) Applicant: BANYU PHARMACEUTICAL CO., LTD., Chuo-ku, Tokyo 103-8416 (JP)
(72) Inventor: AKAO, Atsushi c/o Banyu Pharmaceutical Co., Ltd., Okazaki-shi, Aichi 444-0858 (JP); KAWASAKI, Masashi Banyu Pharmaceutical Co., Ltd., Okazaki-shi, Aichi 444-0858 (JP); KAMATANI, Asayuki, San Diego, CA 92126-5549 (US); MASE, Toshiaki c/o Banyu Pharmaceutical Co., Ltd., Okazaki-shi, Aichi 444-0858 (JP)
(74) Representative: Teipel, Stephan, Dr.
(86) International application number: PCT/JP2003/010672
(87) International publication number: WO 2004/018495

(57) **Abstract**

The present invention provides a process for industrially advantageously producing a compound represented by the formula (I): 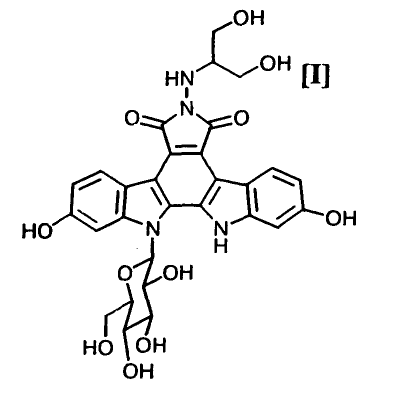 or a pharmaceutically acceptable salt thereof, which is useful as an anticancer agent, and also provides a catalyst used for hydrogenation reaction in the process.

## Description

### Technical Field

The present invention is useful in the field of medicine. More specifically, the present invention relates to a process for industrially advantageously producing a compound which is useful in the filed of medicine.

### Background Art

An indolopyrrolocarbazole derivative produced by the process of the present invention, which is represented by the formula (I): 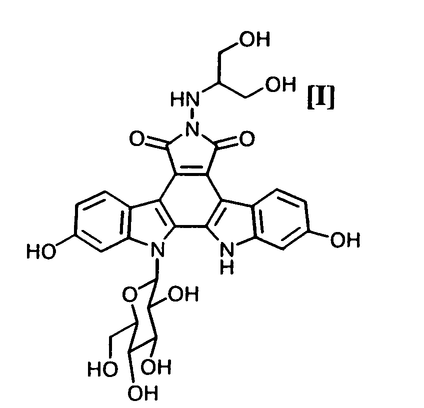 has an anticancer effect, and the compound has now been clinically tested (Mitsuru Ohkubo et al., Bioorganic & Medicinal Chemistry Letters, vol. 9, pages 3307-3312, 1999).

Production processes of the compound of the present invention have been disclosed in WO95/30682 and WO01/62769.

Also, a production process of an indolopyrrolocarbazole derivative represented by the formula (XII): 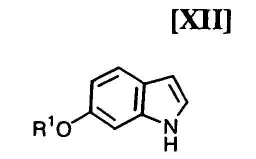 (wherein R¹ represents a hydroxy-protecting group) is disclosed in Organic Synthesis Collective Volumes, vol. 7, page 34.

In addition, a hydrogenation reaction using a rhodium compound, wherein a large amount of iron powders is used as a catalyst for the reduction of the nitro group of a nitrobenzene derivative in an acid solvent such as acetic acid has been known (US-A-5,105,012).

Furthermore, a production process of a bis-indole compound represented by the formula (VIII): 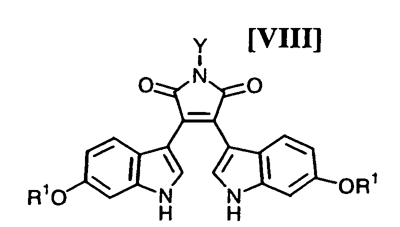 (wherein R¹ represents a hydrogen atom, a C₁-C₇ alkyl group, a phenyl group, a benzyloxymethyl group or an aralkyl group) is disclosed in WO95-30682.

### Disclosure of Invention

An object of the present invention is to eliminate the undesirable aspects in conventional processes for producing an indolopyrrolocarbazole derivative represented by the formula (I) which is useful as a medicine. In other words, an object of the present invention is to provide a production process in which a reagent having high risks in production operations and imposing a heavy environmental burden is not used and a low-yield step is not included.

In a known process for producing an indole compound (Organic synthesis Collective volumes, vol. 7, page 34), a reduction step is conducted using hydrazine in the presence of a Raney nickel catalyst. This process, however, is not desirable for the industrial production, since hydrazine involves a high risk of explosion. Furthermore, since the necessary amount of a Raney nickel catalyst is large, the environmental burden due to post-production waste liquid treatment is heavy, which indicates that this production process is not desirable in the case of industrial mass production.

In addition, the yield is low in known processes for producing a bis-indole compound, and therefore those processes are economically inefficient.

On the other hand, a hydrogenation reaction using a rhodium compound, wherein a large amount of iron powder is used as a catalyst for the reduction of the nitro group of a nitrobenzene derivative in an acidic medium such as acetic acid has been known (US-A-5,105,012). In this case, since hydrogenation reaction is conducted under acidic conditions, this method is not applicable for those materials which are not stable under acidic conditions.

The present inventors had carried out intensive studies on a process for producing an indolopyrrolocarbazole derivative of the formula (I), and found the following (i) to (v).
(i) a novel process for producing an indolopyrrolocarbazole derivative of the formula (I), which has a low environmental burden in terms of waste liquid treatment after production, which is economically excellent, and whose production operation can be carried out safely and with good reproducibility as an industrial production process.
(ii) a safe and novel process for producing an indole derivative of the formula (XII),
(iii) a novel and economically improved process for producing a bis-indole derivative of the formula (VIII),
(iv) a novel hydrogenation catalyst which is safe, has a low environmental burden caused by waste liquid treatment, and can be used not only under an acid condition but also under other conditions, and
(v) a process for producing the compound (VII), in which step control is easy and generation of hydrogen cyanide as a byproduct in the ring-closure reaction using 1,2-dichloro-5,6-dicyano-1,4-benzoquinone can be prevented.

The present inventors conducted further investigations, and finally completed the present invention.

Namely, the present invention relates to a novel process for producing an indolopyrrolocarbazole derivative of the formula (I), a novel process for producing an indole derivative, a novel process for producing a bis-indole derivative and a novel hydrogenation catalyst, which comprise the following (1) to (24).
(1) a process for producing an indolopyrrolocarbazole derivative represented by the formula (I), which comprises the following steps:
   (i): the step of reacting a compound of the formula (XIII) 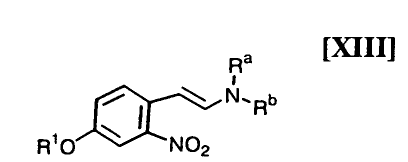 wherein R¹ represents a hydroxy protecting group, and R^{a} and R^{b} each independently represents a C₁-C₇ alkyl group, or R^{a} and R^{b} may be combined together to form a C₃-C₆ alkylenyl group, or a salt thereof with hydrogen gas in the presence of a rhodium compound and a metal compound to produce an indole compound of the formula (XII): 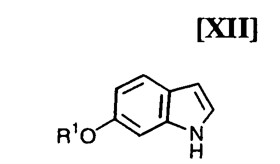 wherein R¹ has the same meaning as defined above, or a salt thereof;
   (ii): the step of reacting the resulting indole compound of the formula (XII) or a salt thereof with a magnesium chloride of the formula (XI):

      R^{c}MgCl [XI]

      wherein R^{c} represents a C₁-C₇ alkyl group, a phenyl group, a vinyl group or an allyl group; or a magnesium compound of the formula (X):

      R^{d}MgR^{d} [X]

      wherein R^{d} represents a C₁-C₇ alkyl group or a phenyl group, or a salt thereof, or a mixture of the magnesium chloride (XI) and the magnesium compound (X), followed by reacting the resulting product with a maleimide compound of the formula (IX): 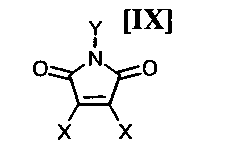 wherein X represents a halogen atom, and Y represents a hydrogen atom, a C₁-C₇ alkyl group, a phenyl group, a benzyloxymethyl group, or a C₇-C₁₂ aralkyl group, to produce a bis-indole compound of the formula (VIII): 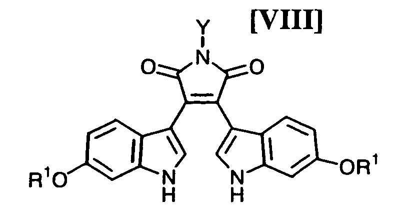 wherein R¹ and Y have each the same meaning as defined above, or a salt thereof;
   (iii): the step of subjecting the resulting bis-indole compound (VIII) or a salt thereof to ring-closure reaction to produce a compound of the formula (VII): 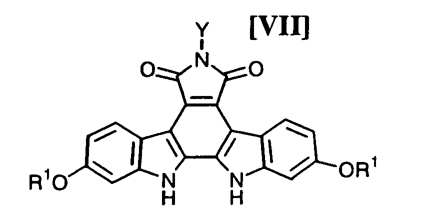 wherein R¹ and Y have each the same meaning as defined above, or a salt thereof;
   (iv): the step of coupling the resulting compound (VII) or a salt thereof with an activated glucose derivative of the formula (VI): 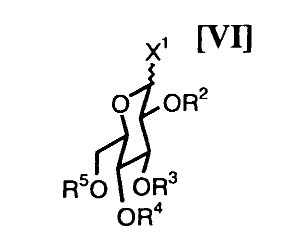 wherein each R², R³, R⁴ and R⁵ is a hydroxy protecting group, and X¹ represents a halogen atom, to produce a compound of the formula (V): 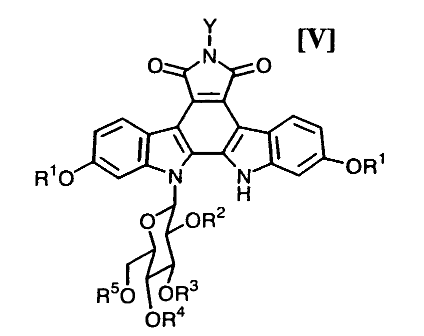 wherein R¹, R², R³, R⁴, R⁵ and Y have each the same meaning as defined above, or a salt thereof;
   (v): the step of treating the resulting compound (V) or a salt thereof with a base to produce a compound of the formula (IV): 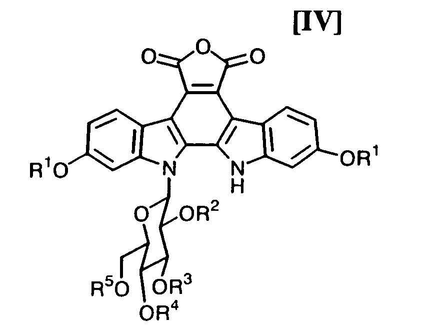 wherein R¹, R², R³, R⁴ and R⁵ have each the same meaning as defined above, or a salt thereof;
   (vi): the step of reacting the compound (IV) or a salt thereof with a compound of the formula (III): 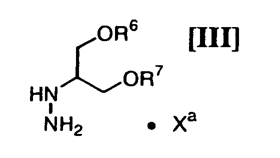 wherein R⁶ and R⁷ each represents a hydroxy protecting group, and X^{a} represents an acid molecule to produce a compound of the formula (II): 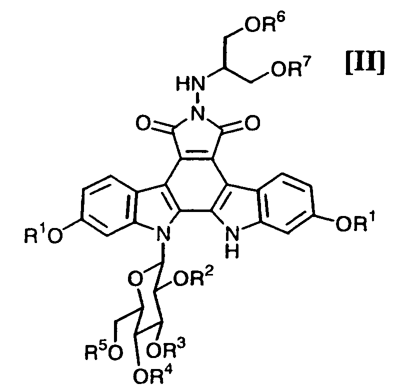 wherein R¹, R², R³, R⁴, R⁵, R⁶ and R⁷ have each the same meaning as defined above, or a salt thereof; and
   (vii): the step of deprotecting the resulting compound (II) or a salt thereof to produce an indolopyrrolocarbazole derivative of the formula (I): 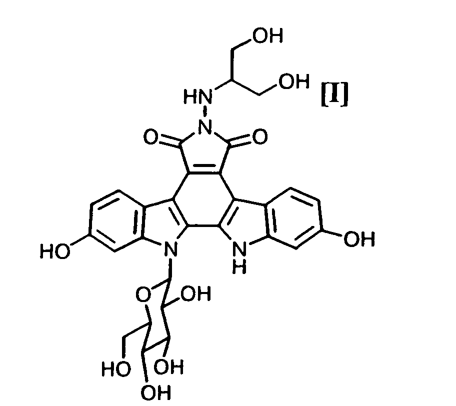 or a salt thereof.
(2) the process according to the above (1), wherein the rhodium compound is rhodium-carbon, rhodium-alumina, rhodium-calcium carbonate or rhodium-barium sulfate;
(3) the process according to the above (1), wherein the metal compound is a nickel(II) compound, an iron(II) compound, an iron(III) compound, a cobalt(II) compound or a cobalt(III) compound;
(4) the process according to the above (3), wherein the nickel(II) compound, the iron(II) compound, the iron(III) compound, the cobalt(II) compound or the cobalt(III) compound are NiBr₂, Ni(NO₃)₂, Ni(OCOCH₃)₂, FeBr₃, FeCl₂ , FeSO₄, FeCl₃, FeCl₃-SiO₂, Fe(OCOCH₃)₂, Fe(II)fumarate, CoBr₂, CoCl₂, 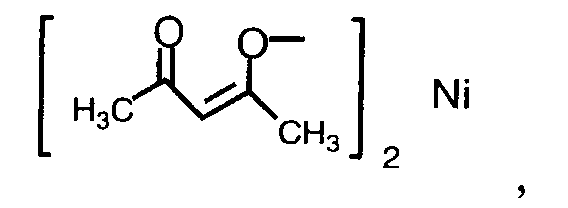 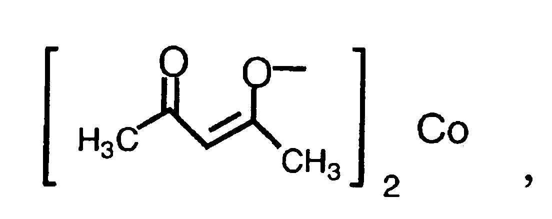 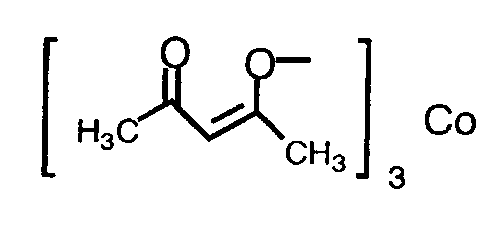 or 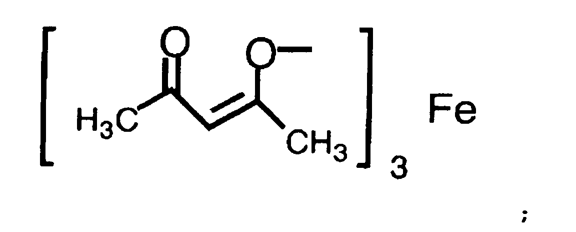
(5) the process according to the above (1), wherein R¹, R², R³, R⁴, R⁵, R⁶ and R⁷ each represents a benzyl group;
(6) the process according to the above (1), wherein the magnesium chloride of the formula (XI) is ethyl magnesium chloride, isopropyl magnesium chloride or n-butyl magnesium chloride;
(7) the process according to the above (1), wherein the magnesium compound of the formula (X) is di(n-butyl)magnesium, di(s-butyl)magnesium, (n-butyl)(s-butyl)magnesium, dimethyl magnesium or diethyl magnesium;
(8) the process according to the above (1), wherein the maleimide compound of the formula (IX) is a maleimide compound represented by the formula (IX-a): 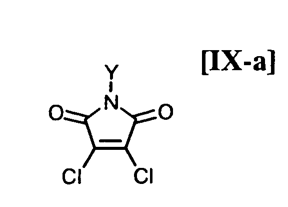 wherein Y represents a hydrogen atom, a C₁-C₇ alkyl group, a phenyl group, a benzyloxymethyl group or an aralkyl group;
(9) the process according to the above (1), wherein Y is a methyl group;
(10) the process according to the above (1), wherein X^{a} is oxalic acid;
(11) the process according to the above (1), wherein the coupling is conducted in the presence of a phase transfer catalyst such as Aliquat 336;
(12) a process for producing an indole compound or a salt thereof, which comprises producing an indole compound represented by the formula (XII): 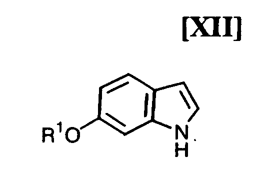 wherein R¹ is a hydroxy protecting group, or a salt thereof by reacting a compound represented by the formula (XIII): 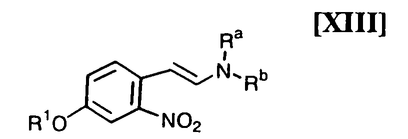 wherein R¹ has the same meaning as defined above, and R^{a} and R^{b} each independently represents a C₁-C₇ alkyl group, or R^{a} and R^{b} may be combined together to form a C₃-C₆ alkylenyl group, with hydrogen gas in the presence of a rhodium compound and a metal compound;
(13) the process according to the above (12), which comprises reacting a compound represented by the formula (XIII): 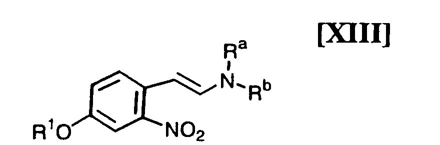 wherein R¹ is a hydroxy protecting group, and R^{a} and R^{b} each independently represents a C₁-C₇ alkyl group, or R^{a} and R^{b} may be combined together to form a C₃-C₆ alkylenyl group, or a salt thereof with hydrogen gas in the presence of a rhodium compound and a metal compound, and treating the resulting crude product with silica gel;
(14) a process for producing a bis-indole compound or a salt thereof, which comprises reacting an indole compound of the formula (XII): 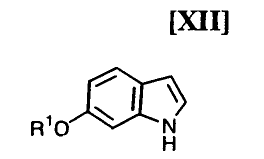 wherein R¹ represents a hydroxy protecting group, or a salt thereof with a magnesium chloride of the formula (XI):

   R^{c}MgCl [XI]

   wherein R^{c} represents a C₁-C₇ alkyl group, a phenyl group, a vinyl group or an allyl group; or a magnesium compound of the formula (X):

   R^{d}MgR^{d} [X]

   wherein R^{d} represents a C₁-C₇ alkyl group or a phenyl group, or a salt thereof, or a mixture of the magnesium chloride of the formula (XI) and the magnesium compound of the formula (X) in an inert solvent, followed by reacting the resulting product with a maleimide compound of the formula (IX): 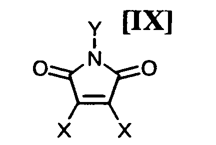 wherein X represents a halogen atom; and Y represents a hydrogen atom, a C₁-C₇ alkyl group, a phenyl group, a benzyloxymethyl group or a C₇-C₁₂ aralkyl group, preferably in an inert solvent to produce a bis-indole compound of the formula (VIII): 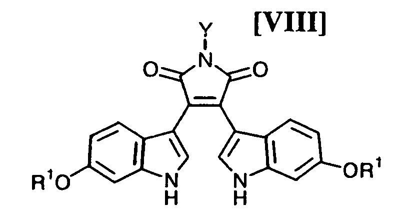 wherein R¹ and Y have each the same meaning as defined above, or a salt thereof;
(15) the process according to the above (14), wherein the maleimide compound of the formula (IX) is a maleimide compound represented by the formula (IX-a): 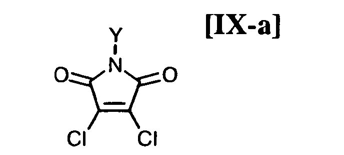 wherein Y represents a hydrogen atom, a C₁-C₇ alkyl group, a phenyl group, a benzyloxymethyl group or a C₇-C₁₂ aralkyl group;
(16) a process for producing a compound represented by the formula (VII): 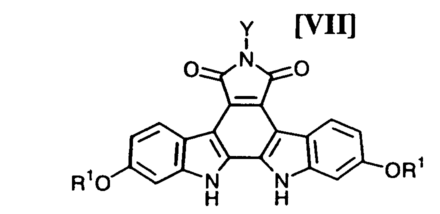 wherein R¹ represents a hydroxy protecting group, and Y represents a hydrogen atom, a C₁-C₇ alkyl group, a phenyl group, a benzyloxymethyl group or a C₇-C₁₂ aralkyl group, or a salt thereof, which comprises treating a bis-indole compound represented by the formula (VIII): 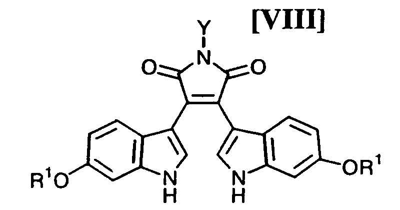 wherein R¹ and Y have each the same meaning as defined above, or a salt thereof with 2,3-dichloro-5,6-dicyano-1,4-benzoquinone in a nonpolar solvent for ring-closure reaction;
(17) the process according to the above (16), wherein the nonpolar solvent is benzene, toluene, xylene (o, m or p), ethylbenzene or 1,2,4-trimethylbenzene;
(18) a catalyst used for hydrogenation reaction, comprising a rhodium compound and a metal compound;
(19) the catalyst according to the above (18), which further comprises an amine;
(20) the catalyst according to the above (18) or (19), wherein the rhodium compound is rhodium-carbon, rhodium-alumina, rhodium-calcium carbonate or rhodium-barium sulfate;
(21) the catalyst according to the above (18) or (19), wherein the metal compound is a nickel(II) compound, an iron(II) compound, an iron(III) compound, a cobalt(II) compound or a cobalt(III) compound;
(22) the catalyst according to the above (19), wherein the amine is a secondary amine or a tertiary amine;
(23) the catalyst according to the above (19), wherein the amine is pyrrolidine, piperidine, dimethylamine, diethylamine, diisopropylamine, dibutylamine, trimethylamine, triethylamine or tributylamine; and
(24) the catalyst according to the above (21), wherein the nickel(II) compound, the iron(II) compound, the iron(III) compound, the cobalt(II) compound or the cobalt(III) compound are NiBr₂, Ni(NO₃)₂, Ni(OCOCH₃)₂, FeBr₃, FeCl₂, FeSO₄ , FeCl₃, FeCl₃-SiO₂, Fe(OCOCH₃)₂, Fe(II)fumarate, CoBr₂, CoCl₂, 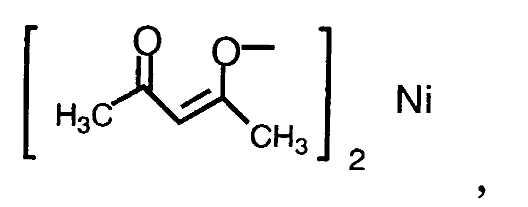 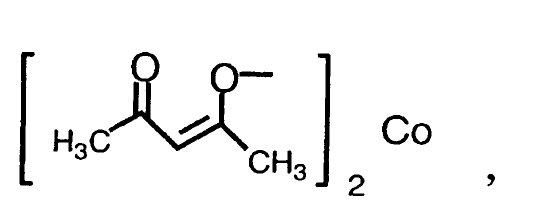 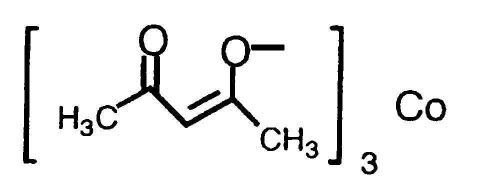 or

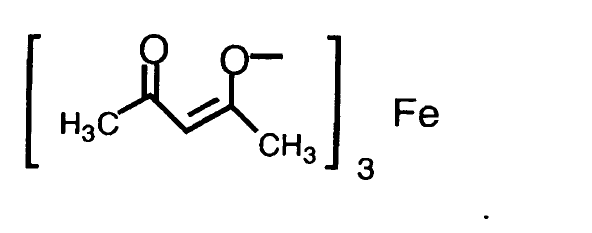

By following the production process of the present invention, it is now possible to industrially produce a compound which is useful as an anticancer agent in the medical field safely, easily and efficiently.

### Best Mode for Carrying Out the Invention

The present invention will be illustrated in more detail below. Firstly, the terms used in the description are explained.

Examples of "C₁-C₇ alkyl group" include a straight or branched alkyl group such as methyl, ethyl, propyl, isopropyl, butyl, isobutyl, t-butyl, n-pentyl, isopentyl, neopentyl, n-hexyl, isohexyl and heptyl, among which methyl, ethyl, propyl, isopropyl or butyl is preferable, and methyl, ethyl, propyl, butyl or heptyl is more preferable.

Examples of "C₃-C₆ alkylene group" include a straight alkylene group such as trimethylene, tetramethylene, pentamethylene and hexamethylene, among which tetramethylene or pentamethylene is preferable.

Examples of "C₇-C₁₂ aralkyl group" include a C₇-C₁₂ aralkyl group such as benzyl, 1-naphtylmethyl and 2-naphtylmethyl, among which benzyl is preferable.

Examples of "acid molecule" include a proton acid such as hydrochloric acid, sulfuric acid, nitric acid, acetic acid, methylsulfonic acid, p-toluenesulfonic acid, oxalic acid, propionic acid, formic acid and benzoic acid, among which oxalic acid is preferable.

Examples of "hydroxy protecting group" include a protecting group for hydroxy groups, such as benzyl, tolyl, p-nitrobenzyl, p-methoxybenzyl and benzyloxymethyl, among which benzyl is preferable.

The "rhodium compound" refers to a catalyst including a rhodium atom, typically a rhodium catalyst supported on a carrier, and preferable examples thereof include rhodium-carbon, rhodium-alumina, rhodium-calcium carbonate or rhodium-barium sulfate.

Examples of "halogen atom" include chlorine, iodine and bromine.

The "metal compound" does not include a rhodium compound and refers to a catalyst which, together with a rhodium compound, promotes the reduction reaction, and examples thereof include a nickel(II) compound, an iron(II) compound, an iron(III) compound, a cobalt(II) compound and a cobalt(III) compound, preferably NiBr₂, Ni(NO₃)₂, Ni(OCOCH₃)₂, FeBr₃, FeCl₂, FeSO₄ , FeCl₃, FeCl₃-SiO₂, Fe(OCOCH₃)₂, Fe(II)fumarate, CoBr₂, CoCl₂, 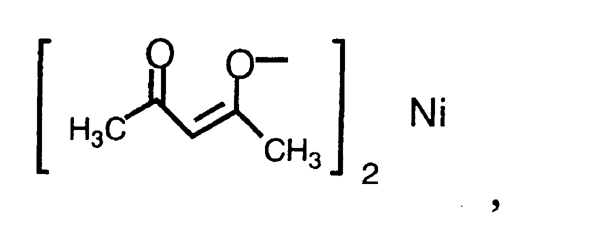 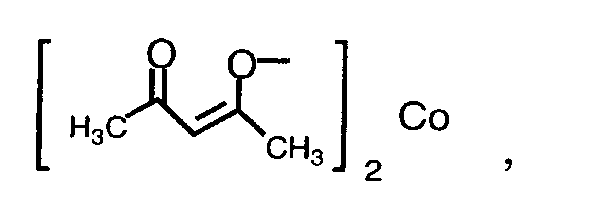 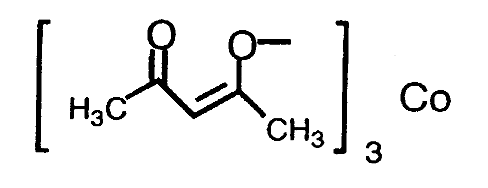 or

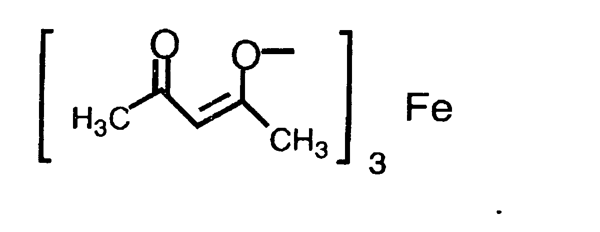

The "phase-transfer catalyst" refers to a catalyst which promotes the reaction between an oleophilic organic compound and a hydrophilic organic compound in a two-phase system consisting of oil phase and aqueous phase, and examples thereof include a compound of the formula (XIV): 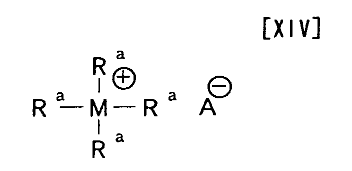 wherein each R^{a} independently represents hydrogen, benzyl or C₁-C₁₈ hydrocarbon; M represents a nitrogen atom or a phosphorous atom; and A represents hydroxy, a fluorine atom, a bromine atom, a chlorine atom, an iodine atom, cyano, HSO₄, CH₃SO₃ or PhCH₂COO, and tris(2-(2-methoxyethoxy)ethyl)amine, and preferable examples thereof include tricaprylmethylammonium chloride, tris(2-(2-methoxyethoxy)ethyl)amine, benzyltriethylammonium chloride and tributylammonium hydrogen sulfate. Specific examples of the compound of the formula (XIV) include tricaprylmethylammonium chloride and the like.

The "salt" typically refers to an acid addition salt, and a pharmaceutically acceptable salt is preferable. Examples of the acid of the acid addition salt include an inorganic acid such as hydrochloric acid and sulfuric acid, and an organic acid such as acetic acid and oxalic acid.

Examples of "amine" include a primary amine, a secondary amine or a tertiary amine, and more specifically include an amine such as pyrrolidine, piperidine, dimethylamine, diethylamine, diisopropylamine, dibutylamine, trimethylamine, triethylamine and tributylamine, preferably a secondary amine or a tertiary amine, and more preferably pyrrolidine.

The "treating with silica gel" refers to the process of filtering the crude product dissolved in a solvent through a column filled with silica gel or a filter whose surface is covered with silica gel.

The preferable production process of the present invention will be illustrated in detail below.

The step of producing an indole compound of the formula (XII): 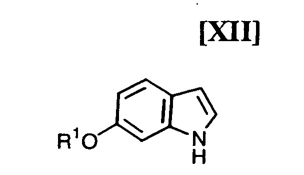 wherein R¹ represents a hydroxy protecting group, by reacting a compound of the formula (XIII): 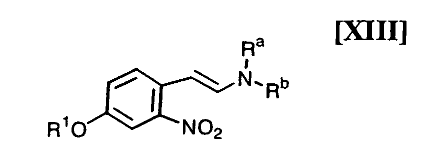 wherein R¹ has the same meaning as defined above, and R^{a} and R^{b} each independently represents a C₁-C₇ alkyl group, or R^{a} and R^{b} may be bonded to each other to form a C₃-C₆ alkylene group, with hydrogen gas in the presence of a rhodium compound and a metal compound can be conducted in such a way that the compound (XIII) is reacted with hydrogen gas at 1 to 5 atm in the presence of about 0.5 mol% to 30 mol% of a rhodium compound and about 1 mol% to 100 mol% of a metal compound, relative to 1 mole of the compound (XIII), in an inert solvent at about -20°C to 80°C for about 1 to 120 hours.

Examples of the inert solvent which may be used in the above step include tetrahydrofuran, diethyl ether, t-butyl methyl ether, diisopropyl ether, dibutyl ether, methanol, ethanol, isopropanol, propanol, acetone, ethyl acetate, isopropyl acetate and cyclopentyl methyl ether, or a mixed solvent thereof, among which tetrahydrofuran, cyclopentyl methyl ether or t-butyl methyl ether is preferable.

The rhodium compound which may be used in the above step may be any compound as long as it has at least one rhodium atom in the molecule, and examples thereof include preferably rhodium-carbon containing 1 to 10% rhodium, rhodium-alumina containing 1 to 10% rhodium, rhodium-calcium carbonate containing 1 to 10% rhodium or rhodium-barium sulfate containing 1-10% rhodium, and more preferably rhodium-carbon.

Examples of the metal compound which may be used in the above step include a nickel(II) compound, an iron(II) compound, an iron(III) compound, a cobalt(II) compound and a cobalt(III) compound, preferably NiBr₂, Ni(NO₃)₂, Ni(OCOCH₃)₂, FeBr₃, FeCl₂, FeSO₄ , FeCl₃, FeCl₃-SiO₂, Fe(OCOCH₃)₂, Fe(II)fumarate, CoBr₂, CoI₂, CoCl₂, 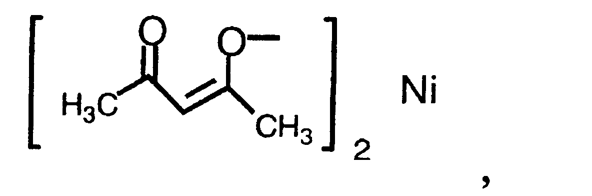 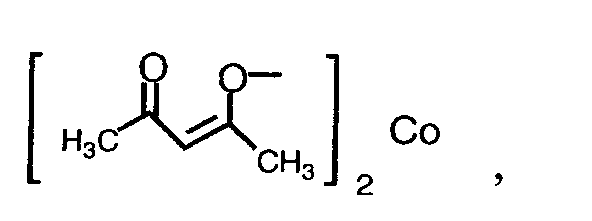 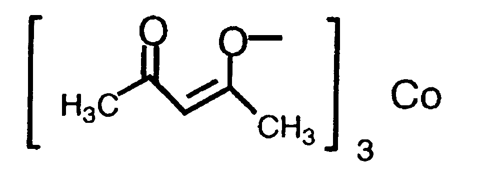 or

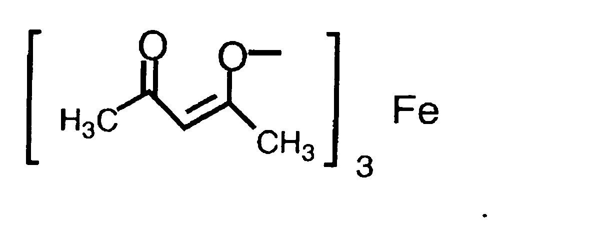

Meanwhile, those starting compounds used in the step can be obtained by a method described in, for example, Organic Synthesis Collective Volumes, vol. 7, page 34, or a similar method thereof.

In the above step, it is preferable to carry out the reaction in the presence of an amine in addition to the rhodium compound and metal compound. Additional presence of an amine in the reaction can improve the reaction rate and the yield. The reaction rate can be dramatically improved with use of an amine. The amine includes a primary amine, a secondary amine or a tertiary amine, and more specifically includes an amine such as pyrrolidine, piperidine, dimethylamine, diethylamine, diisopropylamine, dibutylamine, trimethylamine, triethylamine and tributylamine, preferably a secondary amine or a tertiary amine, and more preferably pyrrolidine.

The amine is usually used in an amount of about 0.01 to 10 equivalents, preferably about 0.01 to 10 equivalents, relative to the material to be hydrogenated (for example, compound (XIII)). Moreover, not by supplementary adding an amine to the reaction solution, but by appropriately selecting a reactant to generate the amine in the reaction solution with the progress of hydrogenation according to the reaction of the present invention, further addition of an amine is not necessary.

Aqueous ammonia and brine are added to the reaction solution (suspension) obtained in this step, preferably a suspension thereof, and the mixture is stirred for about one hour and filtered to separate a solid, and then the residue was washed with a solvent such as benzene, toluene or xylene. The filtrate and the washing are combined and then washed successively with aqueous citric acid, 5% aqueous sodium bicarbonate and brine, and then concentrated in vacuo to dryness. After dissolving the resulting compound of the formula (XII) or a salt thereof in a solvent such as benzene, toluene, xylene and the like, the solution is placed into a column filled with silica gel of the same weight of the compound (XII) or a filter whose surface is covered with the said silica gel, and by applying pressure with an inert gas such as nitrogen. The present inventors found that impurities generated in the reaction step such as colored substances can be effectively removed with the above silica gel purification. The purity of the compound (XII) is improved by the present purification method, and reactions and purification of the products in subsequent steps can be therefore operated in industrially advantageous manners without a special method.

Then, the step of producing a bis-indol compound of the formula (VIII): 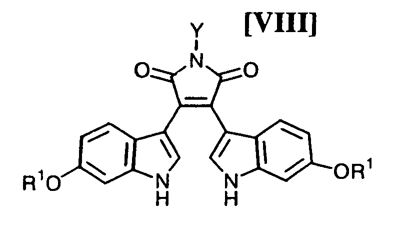 wherein R¹ and Y have each the same meaning as defined above, or a salt thereof by reacting the indole compound (XII): 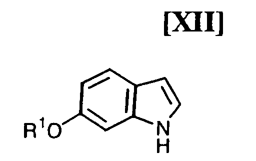 wherein R¹ has the same meaning as defined above, or a salt thereof obtained above with a magnesium chloride of the formula (XI):

R^{c}MgCl [XI]

wherein R^{c} represents a C₁-C₇ alkyl group, a phenyl group, a vinyl group or an allyl group; a magnesium compound of the formula (X):

R^{d}MgR^{d} [X]

wherein R^{d} represents a C₁-C₇ alkyl group or a phenyl group, or a salt thereof, or a mixture of the magnesium chloride (XI) and the magnesium compound (X) in the aforementioned inert solvent, and then reacting the reaction product with a maleimide compound of the formula (IX): 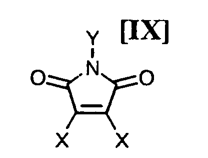 wherein X represents a halogen atom, and Y represents a hydrogen atom, a C₁-C₇ alkyl group, a phenyl group, a benzyloxymethyl group or a C₇-C₁₂ aralkyl group, in the aforementioned inert solvent can be preferably conducted according to the following processes 1), 2) or 3).
1) About 2 to 4 moles of the indol compound (XII) and about 2 to 4 moles of the magnesium chloride (XI), relative to 1 mole of the maleimide compound (IX), are reacted in an inert solvent at about 30°C to 120°C for about 0.5 to 24 hours.
2) About 2 to 4 moles of the indole compound (XII) and about 0.8 to 4 moles of the magnesium compound (X), relative to 1 mole of the maleimide compound (IX), are reacted in an inert solvent at about 30°C to 120°C for about 0.5 to 24 hours.
3) About 2 to 4 moles of the indole compound (XII) and about 0.8 to 4 moles of the mixture comprising the magnesium chloride (XI) and the magnesium compound (X), relative to 1 mole of the maleimide compound (IX), are reacted in an inert solvent at about 30°C to 120°C for about 0.5 to 24 hours.

Preferable examples of the solvent used in the processes 1), 2) and 3) include toluene and a mixture of toluene and tetrahydrofuran.

Examples of the magnesium chloride of the formula (XI) used in the above step include alkyl magnesium chlorides such as methyl magnesium chloride, ethyl magnesium chloride, n-propyl magnesium chloride, isopropyl magnesium chloride, n-butyl magnesium chloride, s-butyl magnesium chloride, isobutyl magnesium chloride, t-butyl magnesium chloride, n-pentyl magnesium chloride, n-hexyl magnesium chloride, phenyl magnesium chloride, vinyl magnesium chloride and allyl magnesium chloride, or a mixture thereof.

Examples of the magnesium compound of the formula (X) used in the above step include dimethylmagnesium, diethylmagnesium, di(n-propyl)magnesium, diisopropylmagnesium, di(n-butyl)magnesium, di(s-butyl)magnesium, diisobutylmagnesium, di(t-butyl)magnesium, di(n-pentyl)magnesium, di(n-hexyl)magnesium, (n-butyl)(s-butyl)magnesium, (methyl)(s-butyl)magnesium, (ethyl)(s-butyl)magnesium, (methyl)(n-butyl)magnesium, (ethyl)(n-butyl)magnesium, (methyl)(t-butyl)magnesium, (ethyl)(t-butyl)magnesium, (n-propyl)(n-butyl)magnesium, (n-propyl)(s-butyl)magnesium, (n-propyl)(i-propyl)magnesium, (n-butyl)(i-propyl)magnesium, (s-butyl)(i-propyl)magnesium, (i-butyl)(i-propyl)magnesium, (n-propyl)(i-butyl)magnesium and diphenylmagnesium, or a mixture thereof.

Then, the step of producing a compound of the formula (VII): 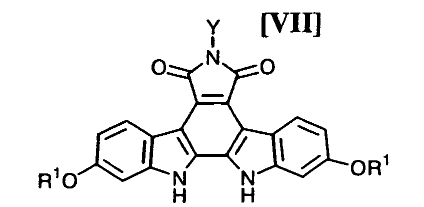 wherein R¹ and Y have each the same meaning as defined above, or a salt thereof by ring-closure reaction of the bis-indole compound (VIII): 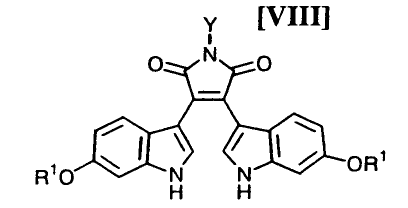 wherein R¹ and Y have each the same meaning as defined above, or a salt thereof obtained above can be conducted preferably according to the following processes 1) and 2).
1) A compound of the formula (VIII) or a salt thereof is treated in an inert solvent with, for example, 2,3-dichloro-5,6-dicyano-1,4-benzoquinone (hereinafter, abbreviated as DDQ), a palladium reagent such as PdCl₂ and Pd(OAc)₂, or a copper reagent such as CuCl₂ in an amount of about 1 to 10 molar equivalents relative to 1 mole of the compound (VIII) or a salt thereof at about 20°C to 200 °C for about 1 minute to 5 days.
   The solvent which may be used in the step 1) may be any solvent as long as it is commonly known as an inert solvent, and examples thereof include polar solvents such as tetrahydrofuran, methanol, ethanol, N,N-dimethylformamide, dimethylsulfoxide, N-methylpyrrolidone and N,N-dimethylacetamide, and a non-polar solvent such as benzene, toluene, xylene (o, m or p), ethylbenzene and 1,2,4-trimethylbenzene. In the case of using 2,3-dichloro-5,6-dicyano-1,4-benzoquinone, hydrogen cyanide is generated during the reaction or the treatment when the above polar solvent is used, while the generation of hydrogen cyanide is suppressed when the non-polar solvent is used, which is advantageous for step control.
2) A compound of the formula (VIII) or a salt thereof is treated with a reagent composed of about 0.01 to 1.0 equivalent of a transition metal catalyst (e.g. palladium, platinum, etc.) supported on carbon, alumina, calcium carbonate, barium sulfate or silica gel, relative to 1 mole of the compound (VIII), in an inert solvent at about 20°C to 200°C for about 1 minute to 5 days under 1 to 5 atm atmosphere of an oxidizing agent selected from the group consisting of oxygen, air, ethylene and acetylene.

Examples of the inert solvent which may be used in the step 2) include toluene, tetrahydrofuran, methanol, ethanol, dimethylformamide, dimethylsulfoxide, N-methylpyrrolidone and dimethylacetamide.

Then, the step of producing the compound of the formula (V): 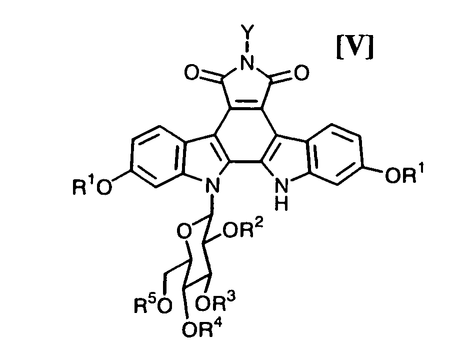 , wherein R¹ and Y have each the same meaning as defined above, and R², R³, R⁴ and R⁵ each represents a hydroxy-protecting group, or a salt thereof by coupling a compound of the formula (VII): 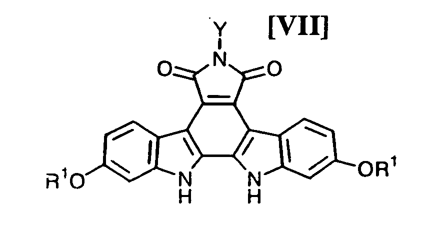 wherein R¹ and Y have each the same meaning as defined above, or a salt thereof obtained above with an activated glucose derivative of the formula (VI): 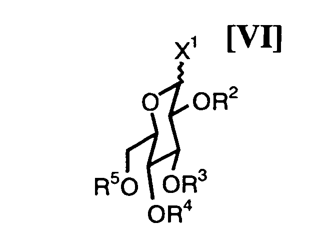 wherein R², R³, R⁴ and R⁵ have each the same meaning as defined above, and X¹ represents a halogen atom, using preferably a system comprising a base in an aqueous solvent and a phase transfer catalyst in an inert organic solvent can be conducted as follows.

The activated glucose derivative (VI): 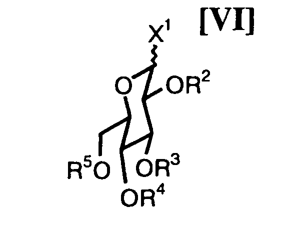 wherein R², R³, R⁴ and R⁵ each represents a hydroxy-protecting group, and X¹ represents a halogen atom, can be produced by reacting a glucose derivative (VIa): 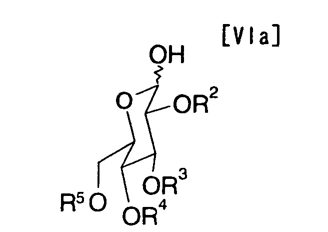 wherein R², R³, R⁴ and R⁵ each represents a hydroxy-protecting group, with, for example, an acid halide, sulfonyl chloride or iodo-triphenylphosphine at about -50°C to 200°C, preferably about -10°C to 30°C, preferably in an inert solvent.

Examples of the acid halide used in the above step include SOCl₂, POCl₃, SOBr₃, POBr₃, PBr₃ and oxalyl chloride, among which SOCl₂ or oxalyl chloride is preferable, and SOCl₂ is more preferable.

Examples of the inert solvent used in the above step include a hydrocarbon such as toluene, xylene, heptane and hexane; a nitrile such as acetonitrile; an ether such as tert-butyl methyl ether and tetrahydrofuran; a halogenated hydrocarbon such as methylene chloride, carbon tetrachloride, chloroform, trifluorotoluene and dichlorobenzene; and a ketone such as methyl isobutyl ketone and acetone, among which tert-butyl methyl ether or tetrahydrofuran is preferable, and tert-butyl methyl ether is more preferable.

As for the glucose derivative of the formula (VIa), commercially available products may be used.

The activated glucose derivative of the formula (VI) obtained above is coupled with a compound of the formula (VII): 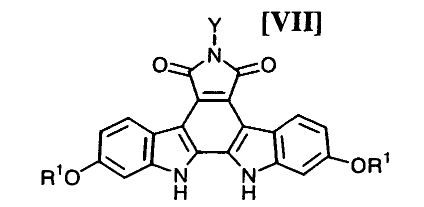 wherein R¹ and Y have each the same meaning as defined above, or a salt thereof, using a system comprising a base in an aqueous solvent and a phase transfer catalyst in an inert organic solvent, usually at about -50°C to 200°C, preferably about 0°C to 40°C.

An example of the aqueous solvent used in the above step is water.

Examples of the base used in the above step include alkali hydroxides such as lithium hydroxide, sodium hydroxide, potassium hydroxide and cesium hydroxide, among which sodium hydroxide or potassium hydroxide is preferable. The concentration of the base used is about 5 wt.% to 95 wt.%, preferably about 45 wt.% to 50 wt.%.

Examples of the inert solvent used in the above step include a hydrocarbon such as toluene, xylene, heptane and hexane; a nitrile such as acetonitrile; an ether such as tert-butyl methyl ether and tetrahydrofuran; a halogenated hydrocarbon such as methylene chloride, carbon tetrachloride, chloroform, trifluorotoluene and dichlorobenzene; a ketone such as methyl isobutyl ketone and acetone; and a non-ionic solvent such as N,N-dimethylformamide and 1-methyl-2-pyrrolidinone, among which tert-butyl methyl ether, methylene chloride or trifluorotoluene is preferable.

Examples of the phase transfer catalyst used in the above step include a compound of the formula (XIV): 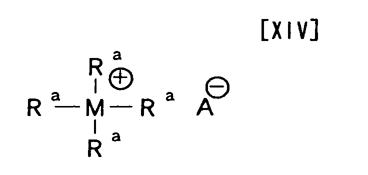 wherein each R^{a} independently represents hydrogen, benzyl or C₁-C₁₈ hydrocarbon; M represents a nitrogen atom or a phosphorous atom; and A represents hydroxy, fluorine, bromine, chlorine, iodine, cyano, HSO₄, CH₃SO₃ or PhCH₂COO, and tris(2-(2-methoxyethoxy)ethyl)amine, and preferable examples thereof include tricaprylmethylammonium chloride, tris(2-(2-methoxyethoxy)ethyl)amine, benzyltriethylammonium chloride and tributylammonium hydrogen sulfate.

The next step of treating the compound of the formula (V) : 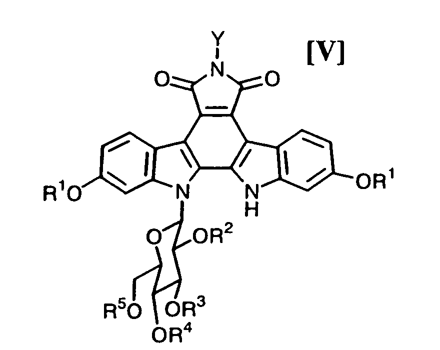 wherein R¹, R², R³, R⁴, R⁵ and Y have each the same meaning as defined above, or a salt thereof obtained above with a base preferably in an inert solvent to produce a compound represented by the formula (IV): 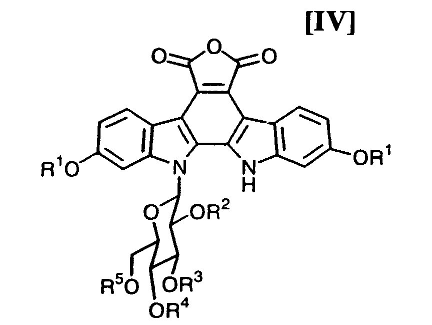 wherein R¹, R², R³, R⁴, and R⁵ have each the same meaning as defined above, or salt thereof is usually carried out using the base in an amount of about 50 to 100 moles, preferably about 50 to 70 moles, relative to 1 mole of the compound (V) or a salt thereof, preferably in an inert solvent which has no adverse effect on the reaction.

Examples of the aforementioned inert solvent include alcohols such as methanol, ethanol, isopropanol and tert-butanol, dimethyl sulfoxide, and a mixed solvent thereof, among which methanol, ethanol or isopropanol is preferable.

Examples of the aforementioned base include a base such as sodium hydroxide, potassium hydroxide, sodium methoxide, potassium methoxide, sodium methoxide, sodium tert-butoxide and potassium tert-butoxide, among which sodium hydroxide, potassium hydroxide or sodium methoxide is preferable.

The reaction temperature is usually from room temperature to about 60°C, preferably about 30°C to 50°C, and the reaction time is usually about 1 hour to 1 day, preferably about 3 hours to 10 hours.

Then, the step of producing a compound of the formula (II): 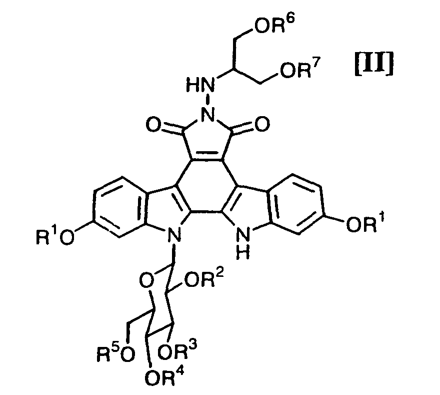 wherein R¹, R², R³, R⁴ and R⁵ have each the same meaning as defined above, and R⁶ and R⁷ each represents a hydroxy-protecting group, or a salt thereof by reacting the compound (IV): 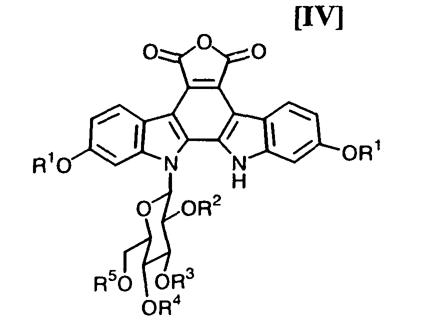 wherein R¹, R², R³, R⁴ and R⁵ have each the same meaning as defined above, or a salt thereof obtained above with the compound (III): 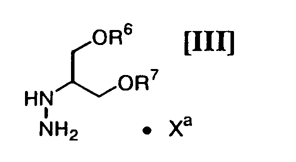 wherein R⁶ and R⁷ have each the same meaning as defined above, and X^{a} represents an acid molecule, is usually carried out using the compound (III) in an amount of about an equivalent mole to 3.0 moles, preferably about 1.0 to 1.5 moles, relative to 1 mole of the compound (IV) or a salt thereof in an inert solvent which has no adverse effect on the reaction.

The said step may be carried out in the presence of an acid scavenger or both an acid scavenger and a desiccant.

The amount of the acid scavenger to be used is about 0.1 to 100 moles, preferably about 0.1 to 2 moles, relative to 1 mole of the compound (IV) or a salt thereof. The amount of the desiccant to be used is about 0.1 to 100 moles, more preferably about 0.1 to 2 moles, relative to 1 mole of the compound (IV) or a salt thereof.

Examples of the aforementioned inert solvent include N,N-dimethylformamide, N,N-dimethylacetamide, tetrahydrofuran, dimethyl sulfoxide and N-methylpyrrolidone, or a mixed solvent thereof, among which N,N-dimethylformamide, N,N-dimethylacetamide or N-methylpyrrolidone is preferable.

The reaction temperature is usually from room temperature to about 90°C, preferably about 30°C to 70°C, and the reaction time is usually about 1 hour to 1 day, preferably about 1 hour to 3 hours.

Examples of the acid scavenger include ethyldimethylamine, triethylamine, isopropyldiethylamine, diisopropylethylamine, tributylamine, pyridine, 2,6-lutidine, 2,6-tert-butylpyridine, 2,4,6-collidine, 1,8-diazabicyclo[5.4.0]non-5-ene (DBU), 1,5-diazabicyclo[4.3.0]undec-7-ene (DBN), diisopropylamine, N,N-dimethylaniline, 1,4-diazabicyclo[2.2.2]octane (DABCO) and N-methylmorpholine, among which lower alkyl amines such as triethylamine, diisopropylethylamine, tributylamine or diisopropylamine is preferable, and triethylamine is more preferable.

Examples of the desiccant include magnesium sulfate, sodium sulfate, the Molecular Sieve, HC(O-i-Pr)₃, HC(O-Et)₃, HC(O-CH₃)₃ and (CH₃)₂C(OCH₃)₂, among which magnesium sulfate, sodium sulfate or the Molecular Sieve is preferable, and magnesium sulfate is more preferable.

Then, in the step of producing an indolopyrrolocarbazole derivative of the formula (I): 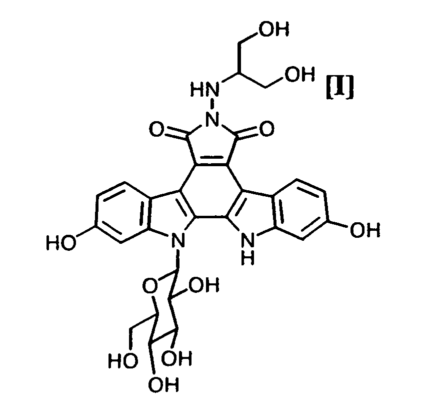 or a salt thereof by deprotection of the compound (II): 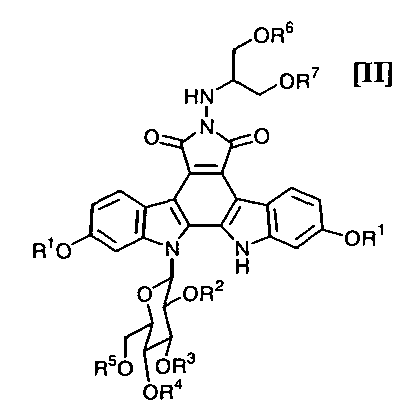 wherein R¹, R², R³, R⁴, R⁵, R⁶ and R⁷ have each the same meaning as defined above, or a salt thereof, when the reaction is carried out by catalytic reduction, the catalyst includes, for example, palladium-carbon catalyst and Raney-nickel catalyst. Such catalysts may be known catalysts.

In the catalytic reduction, preferable hydrogen pressure is usually normal pressure to 3 atm, and the amount of the catalyst used is usually about 1/100 to 1 fold , preferably about 1/100 to 1/10 fold, by mass of the starting compound (II).

Examples of the reaction solvent include mixed solvents of an alcohol solvent, e.g. methanol, ethanol, isopropanol, butanol, and tetrahydrofuran, among which a mixed solvent of isopropanol and tetrahydrofuran (50:50) is preferable.

The reaction temperature is usually from about -30°C to 60°C, preferably about 0°C to 50°C, and the reaction time is usually from an instant to about 7 days, preferably from an instant to about 24 hours.

The method for purification of the obtained compound (I) or a salt thereof may be conducted as follows.

The resulting reaction solution is filtered and the pH of the filtrate is adjusted to about 1.5 to about 6.5, preferably about 1.5 to about 6.5, more preferably about 2.5.

About 10% to about 30%, preferably about 15% to about 25%, more preferably about 20% aqueous alcohol is added to the resulting solution to adjust the concentration of the compound (I) to about 10 mL/g to about 20 mL/g, preferably about 12 mL/g to about 18 mL/g, more preferably about 15 mL/g.

The solution thus obtained is heated to about 50°C to about 100°C, preferably about 70°C.

To the solution, an alcohol in an amount of two-thirds the amount of the solution is added.

The resulting solution is allowed to stand at about 50°C to 100°C, preferably about 70°C, and filtered to collect the precipitated crystals.

In the above filtration, water content of the crystal suspension is adjusted to about 1 to about 10 w/v%.

An example of the alcohol used in the above step includes a C₁-C₅ aliphatic alcohol, preferably methanol, ethanol propanol, isopropanol, butanol, sec-butanol, isobutanol, pentanol and isopentanol, and more preferably isopropanol.

Examples of the base used to adjust the pH include triethylamine, diisopropylethylamine, tributylamine, pyridine, 2,6-lutidine, 2,4,6-collidine, 1,8-diazabicyclo[5.4.0]non-5-ene (DBU), 1,5-diazabicyclo[4.3.0]undec-7-ene (DBN), diisopropylamine, N,N-dimethylaniline, 1,4-diazabicyclo[2.2.2]octane (DABCO) or N-methylmorpholine, among which lower alkyl amines such as triethylamine, diisopropylethylamine, tributylamine and diisopropylamine are preferable, and triethylamine is more preferable.

The compounds obtained by each of the above steps can be purified and isolated, if required, solely or in combination with the methods known per se in the art, such as column chromatography on silica gel or adsorbent resin, liquid chromatography, thin layer chromatography, solvent extraction and recrystallization/reprecipitation.

The present invention also relates to a hydrogenation catalyst comprising the aforementioned rhodium compound and the aforementioned metal compound.

This catalyst is used for the reduction of compounds and contains the aforementioned rhodium compound and metal compound. According to the present invention, coexisting state or mixed state of the rhodium compound and the metal compound is the claimed invention. Therefore, in the catalyst of the present invention, for example, a solvent may be contained or exist in addition to a rhodium compound and a metal compound. The reduction reaction in which the catalyst of the present invention is utilized should not be limited to the reduction reaction in the above (1), while reduction of nitro groups to amino groups, and reduction of alkenyl groups or alkynyl groups to the corresponding alkyl groups are preferable. The catalyst of the present invention has specific actions when it is used in the reduction of nitro groups to amino groups, and reduction of alkenyl groups or alkynyl groups to the corresponding alkyl groups, and it is very useful for industrial purposes. As an example of the said specific actions, a remarkable point is that, in the reduction of nitro groups to amino groups and in the reduction of alkenyl groups or alkynyl groups to the corresponding alkyl groups, even if the material to be reduced has groups other than nitro, alkenyl or alkynyl, such as benzyloxy, carbonyl, e.g. aldehyde or ketone, or halogen in addition to nitro, alkenyl or alkynyl, the reduction of the groups other than nitro, alkenyl or alkynyl is substantially stopped or suppressed, or the reduction of nitro groups to amino groups and the reduction of alkenyl groups or alkynyl groups to the corresponding alkyl groups are predominant over the reduction of the groups other than nitro, alkenyl or alkynyl. Furthermore, the catalyst of the invention also showed that an action of accelerating the reduction of nitro groups to amino groups and the reduction of alkenyl groups or alkynyl groups to the corresponding alkyl groups. When, in particular, catalytic reduction is conducted using a supported rhodium catalyst and a catalyst comprising iron salt, nickel salt or cobalt salt as a metal compound, the reduction of functional groups such as benzyl ether, aryl halides, aldehydes or ketones is substantially stopped or suppressed, and thus the selective reduction of nitro groups to amino groups and the selective reduction of alkenyl groups or alkynyl groups to the corresponding alkyl groups become possible. Consequently, by using the catalyst of the present invention in a reduction reaction, complicated steps taken in conventional reduction reactions involving protection with protecting groups, subsequent reduction procedure and deprotection are not required.

In the present invention, it is preferable that the aforementioned catalyst further contains the aforementioned amine in addition to a rhodium compound and a metal compound, as mentioned above. When the catalyst containing an amine according to the present invention is used, the reaction rate is dramatically accerelated, the reduction rate of functional groups which are easily reduced such as benzyl ether is decreased, and more specific reduction of nitro, alkenyl or alkynyl can be achieved and thus the yield of the reduced product is improved, as compared to the case where the aforementioned catalyst not containing an amine is used.

### Examples

The present invention is described in detail by way of Examples and Reference Examples, however, it is not restricted thereto.

### Example 1

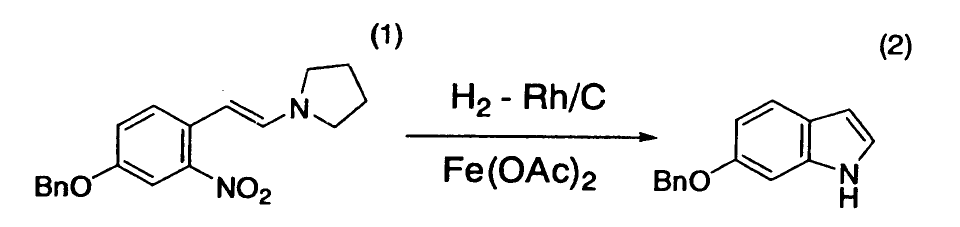

(Bn: benzyl group (hereinafter the same); Rh/C:rhodium carbon powder; Ac: acetyl group)

3-Benzyloxy-6-(2-pyrrolidinylvinyl)nitrobenzene (1) (5.00 g, 15.4 mmol), 5 % rhodium-carbon powder (952 mg, 0.462 mmol), iron(II) acetate (536 mg, 3.08 mmol) and tetrahydrofuran (50 mL) were placed in a 100 mL three-necked flask equipped with a magnetic stirrer and a thermometer under nitrogen atmosphere. The resultant suspension was stirred at 22°C to 25°C for 24 hours under hydrogen atmosphere, and then stirred overnight under nitrogen atmosphere. To the suspension were added 28 % aqueous ammonia (50 g) and 5 % brine (20 mL), and the mixture was stirred for one hour, then filtered to separate a solid. The residual solid was washed with toluene (100 ml), and the previous filtrate and the washing were combined. The solution was washed successively with 10 % aqueous citric acid (50 g), 5 % aqueous sodium bicarbonate (50 g) and 20 % brine (50 g), and concentrated in vacuo to dryness. The residue was dissolved in toluene (about 100 mL) and filtered with a filter covered with silica gel (5 g). The silica gel was washed with toluene and the resultant colorless solution (152.15 g) was analyzed by high performance liquid chromatography, indicating that the objective compound (2) was obtained in 91 % yield (crop 3.15 g).
¹H-NMR (500MHz, DMSO-d₆, δppm): 10.90 (br.s, 1H), 7.49 (d, J= 7.5 Hz, 1H), 7.44 (d, J= 8.6 Hz, 1H), 7.40 (dd, J= 7.5, 7.5 Hz, 1H), 7.33 (dd, J= 7.5, 7.5 Hz, 1H), 7.21 (br.dd, J= 2.4, 2.4 Hz, 1H), 7.01 (br. m, 1H), 6.77 (dd, J= 1.8, 8.6 Hz, 1H), 6.36 (br. m, 1H), 5.12 (s, 2H)
¹³C-NMR (126MHz, DMSO-d₆, δppm): 154.7, 138.0, 136.8, 128.7, 128.0, 127.9, 124.4, 122.5, 120.9, 110.1, 101.3, 96.3, 69.9

### Examples 2 to Examples 18

Similar procedures to that of Example 1 were carried out using rhodium/carbon powder (hereinafter abbreviated as Rh/C) and additives shown in the following table in place of ferrous(II) acetate.

**Table 1**

| Example No. | Amount of Rh/C used | Additives (Amount used) | Reaction time (hr) | Compound (2) Yield |
|---|---|---|---|---|
| 2 | 5 mol % | NiBr₂(10 mol %) | 35 | 79% |
| 4 | 5 mol % | Ni(OAc)₂(20 mol %) | 20 | 87% |
| 5 | 3 mol % | Ni(NO₃)₂(20 mol %) | 24 | 90% |
| 6 | 3 mol % | Ni(acac)₂(20 mol %) | 27 | 86% |
| 8 | 5 mol % | FeCl₃(III)(10 mol %) | 11 | 85% |
| 9 | 5 mol % | FeCl₃(III)/SiO₂(10 mol %) | 18 | 87% |
| 10 | 5 mol % | FeBr₃(III)(20 mol %) | 16 | 78% |
| 13 | 5 mol % | Fe(III)(acac)₃(20 mol %) | 16 | 77% |
| 14 | 3 mol % | FeCl₂(II)(20 mol %) | 21 | 92% |
| 15 | 5 mol % | Fe(II)fumarate (20 mol %) | 16 | 94% |
| 16 | 5 mol % | Fe(II)(acac)₂(20 mol %) | 16 | 86% |
| 17 | 5 mol % | Fe(II)SO₄(20 mol %) | 16 | 96% |
| 18 | 3 mol % | Co(acac)₃(III)(20 mol %) | 12 | 94% |
| Comparison Example 1 | 3 mol % | None | 42 | 62% |

In the table, acac is a group shown by the following formula, and Ac is acetyl.

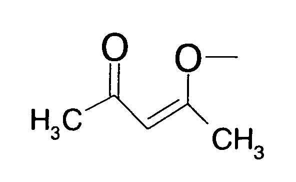

### Example 19

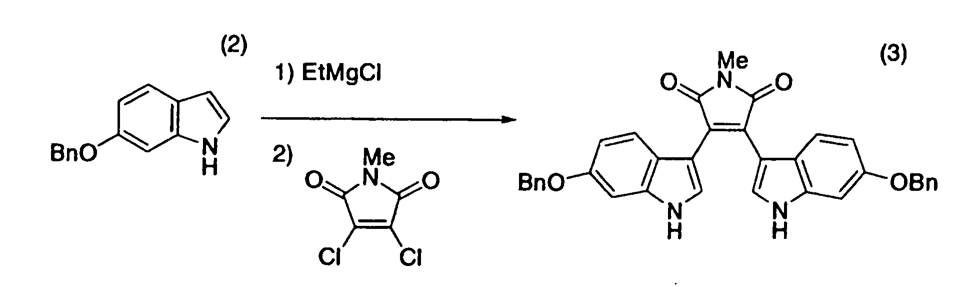

[Et is ethyl and Me is methyl (hereinafter the same)]

6-Benzyloxyindole (2) (2.00 g, 8.96 mmol), tetrahydrofuran (2.70 mL) and toluene (15.2 mL) were placed in a 50 mL three-necked flask equipped with a magnetic stirrer, a Dimroth condenser and a thermometer under nitrogen atmosphere. The mixture was warmed to 33°C, and 2.00 M ethyl magnesium chloride/diethyl ether (4.48 mL, 8.96 mmol) was added over 7 minutes, and the mixture was heated up to 55 °C to 60°C, stirred at 55 °C to 60°C for one hour. N-Methyl-1,2-dichloromaleimide (730 mg, 4.06 mmol) was dissolved in toluene (4.4 mL), and the solution was added to the above mixture over 10 minutes, and the container of the N-methyl-1,2-dichloromaleimide was washed with toluene (1 mL). After addition of the washing, the mixture was stirred at 55 °C to 60°C for 20 minutes. The reaction mixture was further was heated up to 100 °C to 108 °C, stirred at 100°C to 108°C for 12 hours, allowed to stand for cooling to room temperature and stirred overnight. The reaction mixture was heated to 80°C and toluene (15.2 mL) and 13 % aqueous ammonium chloride (17 mL) were added to the mixture. The resultant mixture was cooled down to room temperature to give a suspension, which was filtered to obtain a red solid. The solid was washed successively with toluene (20 mL), toluene-water (mixing ratio of 1:1, 20 mL) and methanol (20 mL x 2). The solid was dried in vacuo overnight at room temperature to give the objective bis-indole (3) in 84 % yield (crop 1.89 g).
¹H-NMR (500MHz, DMSO-d6, δppm): 11.50 (s, 2H), 7.63 (d, J= 2.3 Hz, 2H), 7.42 (d, J= 7.3 Hz, 2H), 7.37 (dd, J= 7.3, 7.3 Hz, 2H), 7.30 (dd, J = 7.3, 7.3 Hz, 2H), 6.97 (d, J= 2.1 Hz, 2H), 6.72 (d, J= 8.8 Hz, 2H), 6.41 (dd, J= 2.1, 8.8 Hz, 2H), 5.04 (s, 4H), 3.03 (s, 3H)
¹³C-NMR (126MHz, DMSO-d6, δppm): 172.2, 155.0, 137.7, 137.1, 128.7, 128.5, 128.1, 128.0, 127.1, 122.0, 120.1, 110.4, 106.1, 96.3, 69.7, 24.3.
M.p. ca. 240°C (decomp.)

### Example 20

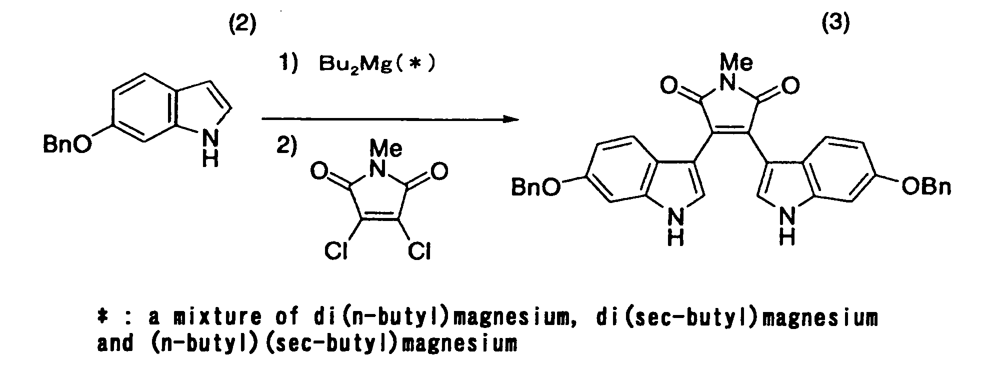

6-Benzyloxyindole (2) (2.00 g, 8.96 mmol), tetrahydrofuran (2.64 mL) and toluene (15.2 mL) were placed in a 50 mL three-necked flask equipped with a magnetic stirrer, a Dimroth condenser and a thermometer under nitrogen atmosphere. The mixture was warmed up to 38°C, and 0.90 M dibutyl magnesium chloride/heptane (4.96 mL, 4.47 mmol) wherein the heptane contained a mixture of di(n-butyl)magnesium, di(sec-butyl)magnesium and (n-butyl)(sec-butyl)magnesium was added over 10 minutes, and the mixture was stirred at 55 °C to 60°C for one hour. N-Methyl-1,2-dichloromaleimide (730 mg, 4.06 mmol) was dissolved in toluene (4.4 mL), and the solution was added to the above mixture over 10 minutes, and the container of the N-methyl-1,2-dichloromaleimide was washed with toluene (1 mL). After addition of the washing, the mixture was heated up to 55 °C to 60 °C, stirred at 55 °C to 60°C to give a solid, which was dissolved homogenously by addition of tetrahydrofuran (1.5 mL). The solution was stirred at 55°C to 60°C for 30 minutes, further heated up to 98 °C to 100 °C, stirred at 98°C to 100°C for 12 hours, allowed to stand for cooling to room temperature and stirred overnight. After the reaction mixture being heated up to 90°C, 13 % aqueous ammonium chloride (17 mL) was added to the reaction mixture, and the mixture was cooled down to room temperature to give a suspension, which was filtered to give a red solid, which was washed successively with toluene (20 mL), toluene-water (mixing ratio of 1:1, 20 mL) and methanol (20 mL x 2). The solid was dried in vacuo overnight at room temperature to give the objective bis-indole (3) in 82 % yield (crop 1.85 g).
¹H-NMR (500MHz, DMSO-d6, δppm): 11.50 (s, 2H), 7.63 (d, J= 2.3 Hz, 2H), 7.42 (d, J=7.3 Hz, 2H), 7.37 (dd, J= 7.3, 7.3 Hz, 2H), 7.30 (dd, J= 7.3, 7.3 Hz, 2H), 6.97 (d, J = 2.1 Hz, 2H), 6.72 (d, J= 8.8 Hz, 2H), 6.41 (dd, J= 2.1, 8.8 Hz, 2H), 5.04 (s, 4H), 3.03 (s, 3H)
¹³C-NMR (126MHz, DMSO-d6, δppm): 172.2, 155.0, 137.7, 137.1, 128.7, 128.5, 128.1, 128.0, 127.1, 122.0, 120.1, 110.4, 106.1, 96.3, 69.7, 24.3
M.p. ca. 240°C (decomp.)

### Comparison Example 2

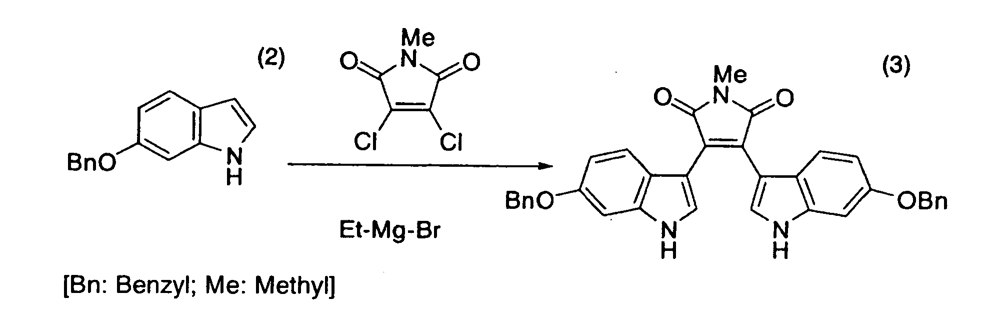

6-Benzyloxyindole (2) (2.00 g, 8.96 mmol), tetrahydrofuran (2.64 mL) and toluene (15.2 mL) were placed in a 50 mL three-necked flask equipped with a magnetic stirrer, a Dimroth condenser and a thermometer under nitrogen atmosphere. The mixture was warmed up to 38°C, and 2.82M ethyl magnesium bromide/diethyl ether (3.12 mL, 8.82 mmol) was added over 7 minutes, heated up to 55 °C to 60 °C, then stirred at 55°C to 60°C for one hour. N-Methyl-1,2-dichloromaleimide (730 mg, 4.06 mmol) was dissolved in toluene (4.4 mL), and the solution was added to the above mixture over 7 minutes, and the container of the N-methyl-1,2-dichloromaleimide was washed with toluene (1 mL). After addition of the washing, the mixture was stirred at 55 °C to 60°C for 30 minutes, further heated up to 100 °C to 107 °C, stirred at 100°C to 107°C for 12 hours, allowed to stand for cooling to room temperature and stirred overnight. After the reaction mixture being heated up to 80°C, toluene (15.2 mL) and 13 % aqueous ammonium chloride (17 mL) were added to the reaction mixture, and the resultant mixture was cooled down to room temperature to give a suspension. The suspension was filtered to give a red solid, which was washed successively with toluene (20 mL), toluene-water (1:1, 20 mL) and methanol (20 mL x 2). The solid was dried in vacuo overnight at room temperature to give the objective bis-indole (3) in 70 % yield (crop 1.73 g).
¹H-NMR (500MHz, DMSO-d6, δppm): 11.50 (s, 2H), 7.63 (d, J= 2.3 Hz, 2H), 7.42 (d, J= 7.3 Hz, 2H), 7.37 (dd, J= 7.3, 7.3 Hz, 2H), 7.30 (dd, J= 7.3, 7.3 Hz, 2H), 6.97 (d, J= 2.1 Hz, 2H), 6.72 (d, J= 8.8 Hz, 2H), 6.41 (dd, J= 2.1, 8.8 Hz, 2H), 5.04 (s, 4H), 3.03 (s, 3H)
¹³C -NMR (126MHz, DMSO-d6, δppm): 172.2, 155.0, 137.7, 137.1, 128.7, 128.5, 128.1, 128.0, 127.1, 122.0, 120.1, 110.4, 106.1, 96.3, 69.7, 24.3
M.p. ca. 240°C (decomp.)

### Example 21

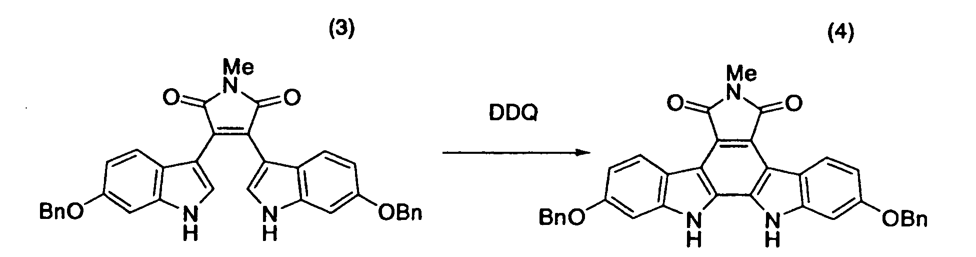

Bis-indole compound (3) (3.00 g, 5.42 mmol) and toluene (75.3 mL) were placed in a 300 mL three-necked flask equipped with a magnetic stirrer, a Dimroth condenser and a thermometer under nitrogen atmosphere, and the mixture was heated up to 110°C. A solution of 2,3-dichloro-5,6-dicyano-1,4-benzoquinone (DDQ)(1.37 g, 6.03 mmol) in toluene (48.0 mL) was added to the above mixture over 15 minutes at 107°C to 110°C. After washing the container containing DDQ with toluene (12 mL), the washing is also added to the reaction mixture. The mixture was stirred at 108°C to 110°C for one hour, and analyzed by high performance liquid chromatography, indicating that the starting material disappeared. The reaction solution was cooled to 71°C and methanol (134 mL) was added over 3 hours at 60°C to 71°C [measurement stage:A]. The mixture was cooled down to room temperature and stirred overnight [measurement stage: B]. The reaction solution was filtered and washed with methanol (15 mL x 2) to give a brown solid (2876 mg), which was suspended in N,N-dimethylformamide (54 mL). The suspension was heated and stirred at 95°C to 105°C for one hour, cooled down to room temperature and stirred overnight. The reaction solution was filtered and washed with methanol (15 mL x 2) to give a yellow solid (3018 mg), which was suspended in dimethyl sulfoxide (28.3 mL). The suspension was heated to 60°C to 70°C to dissolve the above solid. Methanol (13.3 mL) and a small amount of the above-identified compound as a seed crystal were successively added, and the mixture was stirred for one hour to mature the suspension. After addition of methanol (42.4 mL) over a period of 2 hours, the mixture was cooled down to room temperature and then stirred overnight. The reaction mixture was filtered , washed with methanol (15 mL x 2), dried in vacuo at 60°C overnight to give the objective indolocarbazole derivative (4) as yellow crystals in 87 % yield (crop 2589 mg).
¹H-NMR (500MHz, DMSO-d₆, δppm): 11.26 (s, 2H), 8.69 (d, J= 8.7 Hz, 2H), 7.54 (d, J= 7.3 Hz, 2H), 7.43 (dd, J= 7.3, 7.3 Hz, 2H), 7.37 (dd, J= 7.3, 7.3 Hz, 2H), 7.27 (d, J= 2.1 Hz, 2H), 6.72 (d, J= 8.8 Hz, 2H), 6.96 (dd, J= 2.1, 8.7 Hz, 2H), 5.22 (s, 4H), 2.96 (s, 3H).
M.p. ca. 324°C (decomp.)
HPLC measurement conditions:
Separation column YMC AM-303 250 x 4.6 mm, 40°C,
UV=220 nM, Injection amount 10 µL,
Mobile phase: MeCN-0.1 % phosphoric acid = (t=0, 65:35; t=20, 90:30), Flow rate 1 mL/min.

### Example 22

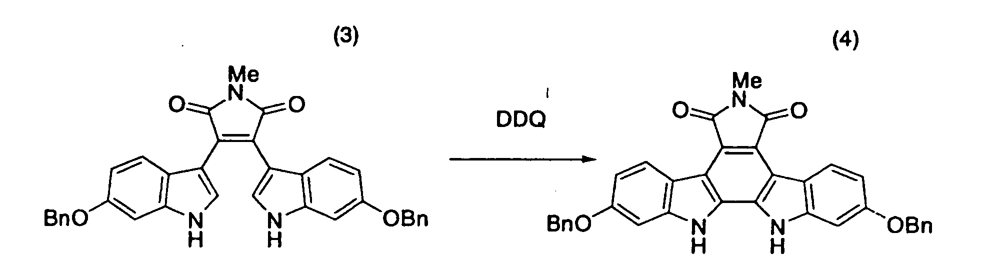

Toluene (75.3 mL) and the bis-indole compound (3)(3.00 g, 5.42 mmol) were placed in a 300 mL four-necked flask under nitrogen atmosphere, and the resultant suspension was heated to 70°C. To the suspension was added a solution of 2,3-dichloro-5,6- dicyano-1,4-benzoquinone (DDQ) (1.29 g, 5.69 mmol) in N,N- dimethylformamide (24.0 mL) over one hour. After washing the container which contained DDQ with N, N-dimethylformamide ( 6 . 0 mL), the washing is also added to the reaction mixture. The mixture was stirred at 70°C for one hour [measurement stage:C], and analyzed by high performance liquid chromatography, indicating that the starting material disappeared. After addition of methanol (134 mL) over 2 hours, the mixture was stirred at 70°C for one hour and cooled down to 25°C and further stirred at the same temperature overnight. The reaction mixture was filtered and washed with methanol (15 mL x 2) to give a yellow solid, which was dried in vacuo overnight at 25°C to give the objective indolocarbazole derivative (4) as crude yellow crystals (3.08 g).

### Example 23

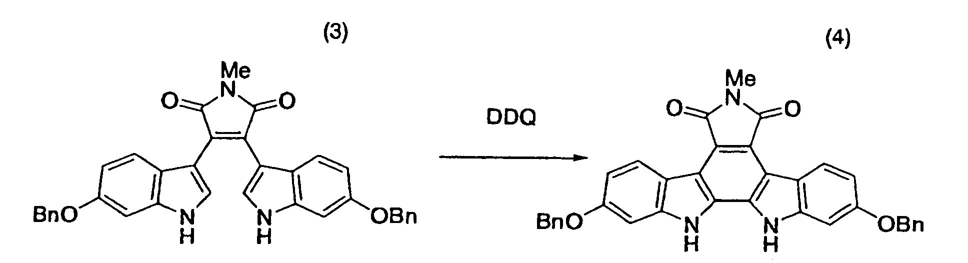

Toluene (28.6 kg) and the bis-indole compound (3) (1.50 kg, 2.71 mol) were placed in an 80 L reaction vessel under nitrogen atmosphere, and the inner wall of the vessel was washed with toluene (3.9 kg). After addition of the washing, the resultant suspension was heated to 110°C, and a solution of 2,3-dichloro-5,6-dicyano-1,4-benzoquinone (DDQ) (0.65 kg, 2.86 mol) in toluene (20.8 L) was added to the above suspension over one hour. After washing the container, in which DDQ was contained, with toluene (5.2 kg), the washing is also added to the reaction solution. The mixture was stirred at 110°C for one hour and analyzed by high performance liquid chromatography, indicating that the starting material disappeared. The mixture was cooled down to 25°C over 0.5 hour [measurement stage D] and stirred at the same temperature for one hour. The reaction mixture was filtered and washed with toluene (13 kg) to give a brown solid (2.07 kg), which was dried in vacuo overnight at 60°C. The solid was suspended in N,N-dimethylformamide (25.4 kg) and the suspension was stirred at 100°C to 105°C for one hour. The suspension was cooled down to 25°C over a period of one hour and stirred overnight [measurement stage E]. The reaction mixture was filtered, washed with N,N-dimethylformamide (9.8 kg) and methanol (8.2 kg), and dried overnight at 60°C to give a yellow solid (1.51 kg). The yellow solid was suspended in dimethyl sulfoxide (16.7 kg). The suspension was heated to 60°C to dissolve the above solid. Methanol (5.6 kg) and a seed crystal (8.0 g) of the objective indolocarbazole derivative (4) were successively added to the suspension. The suspension was stirred at 60°C to 65°C for one hour for maturing crystal formation. Further, after addition of methanol (18.0 kg) over a period of 2 hours, the mixture was stirred at the same temperature for one hour, cooled down to 25°C and stirred overnight. The reaction solution was filtered, washed with methanol (12 kg) and dried in vacuo overnight at 60°C to give the objective indolocarbazole derivative (4) as yellow crystals (1.29 kg, yield 86 %). The measurement stage in the parentheses means a stage when hydrogen cyanide was measured, and hydrogen cyanide measurement as shown in the following Test Examples is to be referred.

NMR spectrum and IR spectrum of the objective compound (4) in this Example agreed to those of the objective compound of Example 21.

### Test Examples

**Table 2:**

| Measurement of hydrogen cyanide | | |
|---|---|---|
| Example No. | Measurement stage | Cyanide ion content (ppm) |
| 21 | A | 50 |
| 21 | B | 100 |
| 22 | C | 80 |
| 23 | D, E | Not Detected |

### Measurement method:

Nitrogen gas discharged from the reaction vessel was introduced into 0.05 N sodium hydroxide solution (7 ml of 0.05 N sodium hydroxide solution was used to 1 g of the bis-indole compound (3)) during the treatment. With respect to the resultant sodium hydroxide solution, cyanide ion was measured using an ion test paper (CN⁻) available from ADVANTEC.

### Example 24

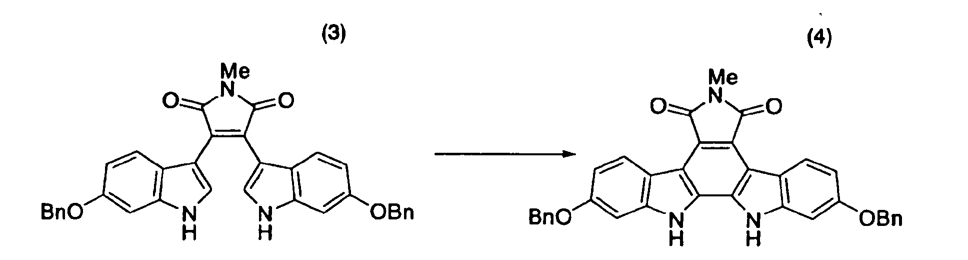

The bis-indole compound (3) (500 mg, 0.903 mmol), 5 % palladium-carbon powder (384 mg, 0.181 mmol) and toluene (22 mL) were placed in a 50 mL eggplant type flask equipped with a magnetic stirrer, under air atmosphere. The mixture was heated in an oil bath of 105°C, stirred at the same temperature for 4 days, cooled and concentrated to dryness. After addition of dimethyl sulfoxide (40 mL) to the residue, insoluble materials were removed by filtration. The filtrate was analyzed by high performance liquid chromatography, indicating that the objective compound (4) as a dimethyl sulfoxide solution was obtained in yield 66 % (330 mg).

NMR spectrum and IR spectrum of the objective compound (4) in this Example agreed to those of the objective compound of Example 21.

### Example 25

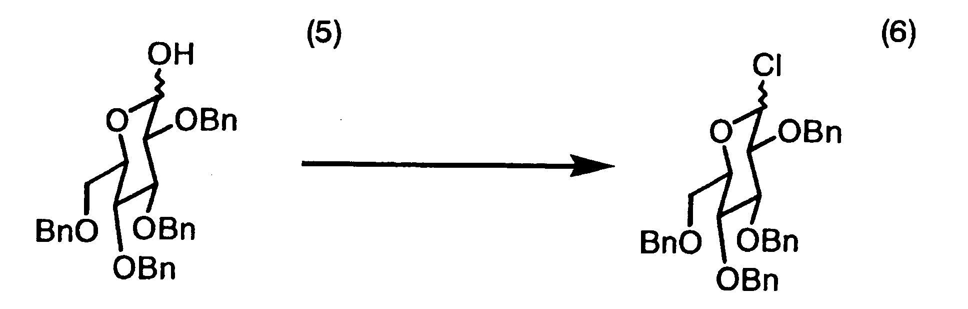

2,3,4,6-O-Tetrabenzyl-D-glucopyranose (5) (100.00 g, 185 mmol) was dissolved in N,N-dimethylformaldehyde (360 mL) at 23°C. The solution was cooled to 9°C and thionyl chloride (16.2 mL, 222 mmol) was added thereto over a period of 15 minutes, whereby the temperature was raised to 20°C. The resultant solution was warmed to 30°C and allowed to stand for one hour. The solution was cooled to -10°C, and 10 % (w/w) potassium hydroxide solution (about 150 mL) was added while maintaining the temperature of not higher than 0°C. The solution was warmed to 22°C and separated into an organic layer and an aqueous layer. The aqueous layer was extracted with tert-butyl methyl ether (300 mL x 1). The previous organic layer and the tert-butyl methyl ether extract were combined, and the solution was washed successively with saturated brine (150 mL x 1) and water (200 mL x 1). The solution was concentrated in vacuo to a volume of 350 mL containing 1-chloro-2,3,4,6-tetrabenzyl-1-deoxy-D-glucopyranose (6). The concentrate was served as a starting material for Example 26 without purification.

### Example 26

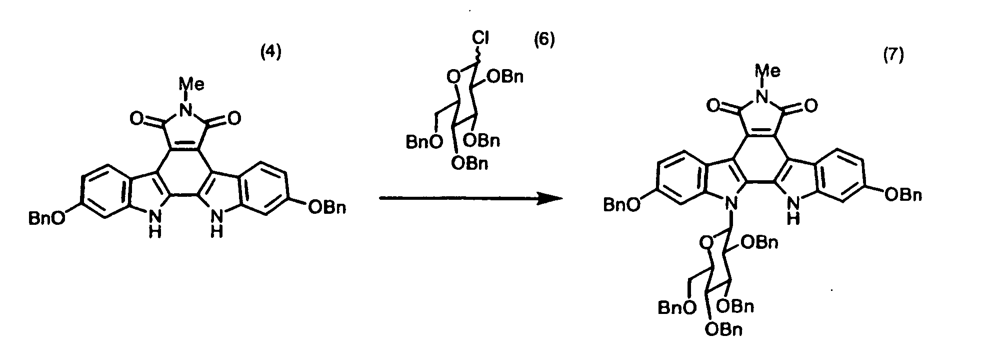

The indolocarbazole derivative (4) (72.00 g, 131 mmol), which was obtained in Example 21, was dissolved in tert-butyl methyl ether (600 mL). The solution was stirred at 23°C for 10 minutes, and to this solution was added a solution (350 mL) containing 1-chloro-2,3,4,6-tetrabenzyl-1-deoxy-D-glucopyranose (6) obtained in Example 25. The solution was stirred for 10 minutes, and 45 % (w/w) potassium hydroxide solution (300 mL) was added. The solution was stirred for 10 minutes, and 40 % (w/w) Aliquat (Registered Trade Mark) 336 (Product Name) in tert-butyl methyl ether (obtained by dissolving Aliquat 336 (72 g) in tert-butyl methyl ether (110 g)) was added gradually over 22 minutes. The mixture was stirred at 23°C for 6 hours, and water (350 mL) was added thereto. The mixture was stirred for 5 minutes, and separated into an organic layer and an aqueous layer. The aqueous layer was washed with tert-butyl methyl ether (300 mL x 1). The tert-butyl methyl ether layer and the organic layer were combined, washed with 10 % (w/w) aqueous citric acid (300 mL x 1) and then with water (300 mL x 1). The organic solution was stirred overnight at 22°C to precipitate crystals of the objective indolocarbazole derivative (7). The resultant suspension was concentrated to a volume of about 625 mL under atmospheric pressure, and cooled to 23°C. Methanol (225 mL) was added gradually to the suspension over one hour, and the suspension was cooled to -5°C and stirred for 45 minutes to give crystals. The crystals were collected by filtration, washed with cold methanol/tert-butyl methyl ether (1:1) (400 mL x 2) and dried in vacuo at 25°C to 40°C.

Analysis of the resultant crystals by high performance liquid chromatography indicated that the content of the objective indolocarbazole derivative (7) was 99 %.

The Aliquat (Registered Trade Mark) 336, available from Aldrich Chemical Co., Inc.) used in this Example is tricaprylmethylammonium chloride.

### Example 27

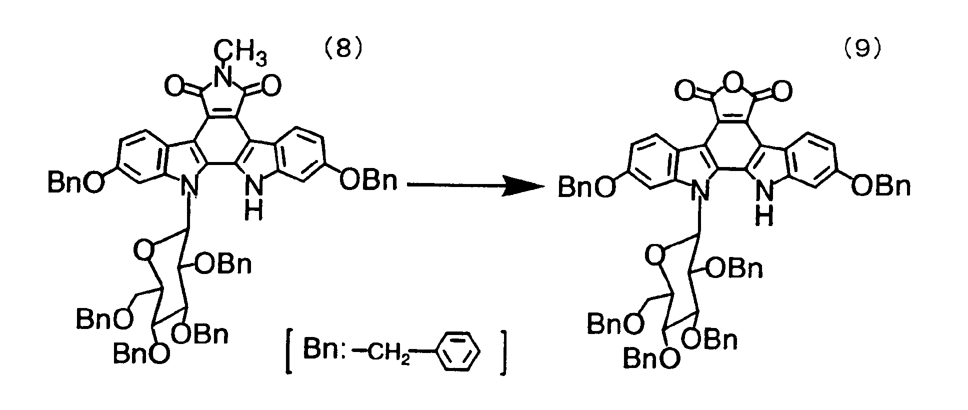

Ethanol (36 mL) was added to a 300 mL four-necked flask equipped with a stirrer and a thermometer, and 12,13-dihydro-2,10-dibenzyloxy-13-(B-D-2,3,4,6-tetra-O-benzylglucopyranosyl)-5H-indolo[2,3-a]pyrrolo[3,4-c]carbazole-6-methyl-5,7(6H)-dione (8)(670 mg, 0.62 mmol) was added thereto while stirring. The mixture was stirred at room temperature for one hour, and 5N aqueous potassium hydroxide (8 mL) was added dropwise at the same temperature over 20 minutes. The mixture was stirred at an inner temperature of 60°C for 4 hours and at room temperature overnight to give a brown solution, to which was added toluene (20 mL). After dropwise addition of 1.0 N hydrochloric acid (62 mL) at the same temperature over 30 minutes to adjust the pH to 2.6, a yellow solution was obtained. Tetrahydrofuran (10 mL) was added thereto and the mixture was stirred for 6 hours. The aqueous layer (the lower layer) was separated, and the organic layer was washed successively with purified water (10 mL x 2) and saturated brine (10 mL), dried over anhydrous sodium sulfate (5 g) and filtered. The filtrate was concentrated in vacuo to give 12,13-dihydro-2,10-dibenzyloxy-13-(β-D-2,3,4,6-tetra-O-benzylglucopyranosyl)-5H-indolo[2,3-a]carbazole-5,6-dicarboxylic anhydride (0.63 g) as a yellow oily residue in 85 % yield.
¹H-NMR (270MHz, CDCl₃), δ(ppm): 10.79 (1H, s), 9.04 (1H, d, J=9.2Hz), 8.95 (1H, d, J=9.6Hz), 7.26 (32H, m), 6.17 (2H, d, J=7.3Hz), 5.85 (1H, d , J=8.2Hz), 4.89 (10H, m), 4.32(1H, t, J=8.9Hz), 3.96 (6H, m), 3.13 (1H , d, J=10.2Hz)

### Example 28

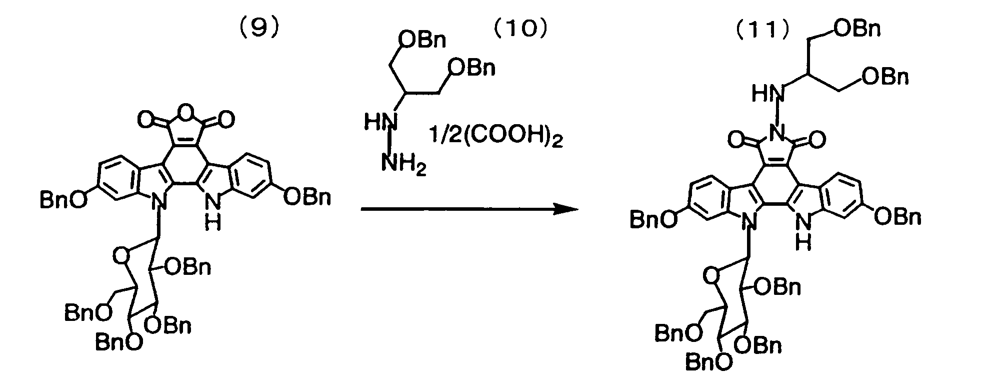

A mixture of 12,13-dihydro-2,10-dibenzyloxy-13-(β-D-2,3,4,6-tetra-O-benzylglucopyranosyl)-5H-indolo[2,3-a]carbazole-5,6-dicarboxylic anhydride (9) (1.50 g, 1.41 mmol) obtained in Example 27, N-(1-benzyloxymethyl-2-benzyloxyethyl)hydrazine hemioxalate (10) (609 mg, 1.84 mmol) and N,N-dimethylacetamide (14 mL) was degassed and the atmosphere was substituted for nitrogen. The mixture was heated to 62°C, and triethylamine (0.26 mL, 1.84 mmol) was added dropwise. After stirring at the same temperature for 3 hours, the reaction mixture was cooled down to room temperature, and methyl tert-butyl ether (10 mL) and water (7 mL) were added thereto. The organic layer was separated, washed with water, dried over sodium sulfate and filtered. The solvent was removed by evaporation in vacuo to give the objective compound (11).
¹H-NMR (270MHz, CDCl₃, δppm): 10.63 (1H, br.s), 9.24 (1H, br.d, J=9.6Hz), 9.16 (1H, br.d, J=9.6Hz), 7.50-6.84 (42H, m), 6.20 (2H, br.d, J=7.6Hz), 5.84 (1H, d, J=8.6Hz), 5.33 (1H, br.d, J=3.0Hz), 5.21 (1H, d, J=12.2Hz), 5.19 (1H, d, J=11.9Hz), 5.16 (1H, d, J=12.2Hz), 5.08 (1H, d, J=11.9Hz), 5.08 (1H, d, J=10.9Hz), 4.96 (1H, d, J=10.9Hz), 4.89 (1H, d, J=10.9Hz), 4.85 (1H, d, J=10.9Hz), 4.72 (1H, d, J=12.9Hz), 4.68 (1H, d, J=12.9Hz), 4.62-4.48 (4H, m), 4.33 (1H, dd, J=9.6, 9.6Hz), 4.06-3.77 (7H, m), 3.72 (4H, d, J=5.6Hz), 3.04 (1H, d, J=9.9Hz) ¹³C-NMR (68MHz, CDCl₃, δppm): 168.8, 168.7, 159.4, 159.3, 143.2, 142.9, 138.0, 137.9, 137.6, 136.9, 136.8, 136.6, 136.0, 130.2, 128.7, 128.6, 128.5, 128.4, 128.3, 128.2, 128.2, 128.1, 128.0, 127.9, 127.8, 127.7, 127.6, 127.5, 127.4, 127.3, 126.9, 126.6, 119.4, 119.1, 118.0, 116.9, 116.7, 116.1, 110.4, 96.7, 96.3, 85.8, 84.7, 80.9, 77.4, 77.2, 76.0, 75.9, 75.4, 74.9, 73.9, 73.3, 73.2, 70.7, 70.4, 69.9, 69.8, 66.7, 58.7, 49,4, 30.9, 27.0

### Example 29

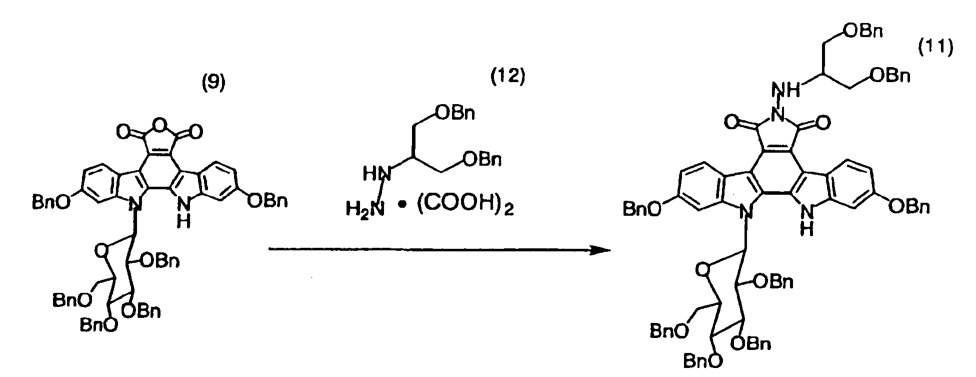

A mixture of 12,13-dihydro-2,10-dibenzyloxy-13-(β-D-2,3,4,6-tetra-O-benzylglucopyranosyl)-5H-indolo[2,3-a]carbazole-5,6-dicarboxylic anhydride (9)(1.30 g, 1.23 mmol) obtained in Example 27, N-(1-benzyloxymethyl-2-benzyloxyethyl)hydrazine monooxalate (599 mg, 1.59 mmol) and N,N-dimethylacetamide (12.3 mL) were degassed. The mixture was heated to 45°C under nitrogen atmosphere, and triethylamine (34.1 µL, 0.25 mmol) was dropwise added thereto. The mixture was stirred at the same temperature for 16 hours, and cooled down to room temperature. After addition of methyl tert-butyl ether (25 mL) and water (6.1 mL), the organic layer was separated, washed four times with water (5.2 mL x 4), dried over magnesium sulfate and filtered. The filtrate was analyzed by high performance liquid chromatography, indicating that the objective compound (11) (1.50 g) was obtained as a solution in 92 % yield.

Since NMR spectrum and IR spectrum of the objective compound (11) in this Example agreed to those of the objective compound of Example 28, the compound (11) was identified as 12,13-dihydro-2,10-dibenzyloxy-6-N-(1-benzyloxymethyl-2-benzyloxyethylamino)-13-(β-D-2,3,4,6-tetra-O-benzylglucopyranosyl)-5H-indolo[2,3-a]pyrrolo[3,4-c]carbazole--5,7(6H)-dione.

### Example 30

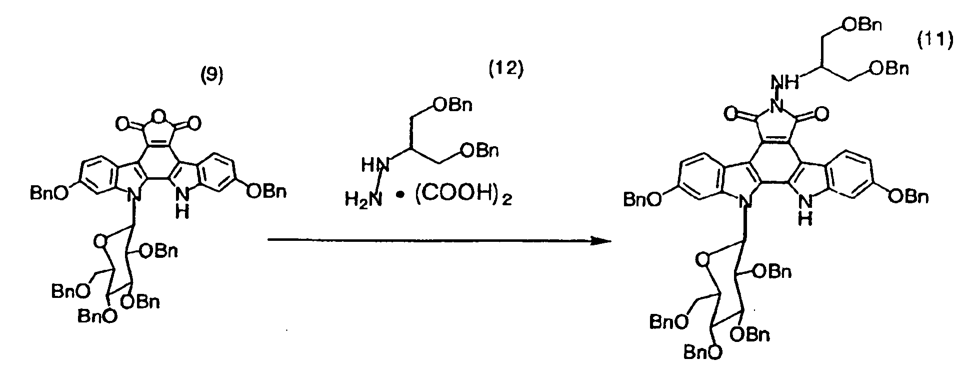

A mixture of 12,13-dihydro-2,10-dibenzyloxy-13-(β-D-2,3,4,6-tetra-O-benzylglucopyranosyl)-5H-indolo[2,3-a]carbazole-5,6-dicarboxylic anhydride (9) (1.30 g, 1. 2 3 mmol) obtained in Example 27, N-(1-benzyloxymethyl-2-benzyloxyethyl)hydrazine monooxalate (599 mg, 1.59 mmol), magnesium sulfate (1.48 g, 12.3 mmol) and N,N-dimethylacetamide (12.3 mL) were degassed. The mixture was heated to 45°C under nitrogen atmosphere, and triethylamine (446 µL, 3.20 mmol) was dropwise added thereto. The mixture was stirred at the same temperature for 10 hours, and cooled down to room temperature. After addition of methyl tert-butyl ether (25 mL) and water (6.1 mL), the aqueous layer was adjusted to pH 3.5 with 2N HCl (1.34 mL). The organic layer was separated, washed four times with water (5.2 mL), dried over magnesium sulfate and filtered. The filtrate was analyzed by high performance liquid chromatography, indicating that the objective compound (11) (1.50 g) was obtained as a solution in 92 % yield.

Since NMR spectrum and IR spectrum of the objective compound (11) in this Example agreed to those of the objective compound of Example 28, the compound (11) was identified as 12,13-dihydro-2,10-dibenzyloxy-6-N-(1-benzyloxymethyl-2-benzyloxyethylamino)-13-(β-D-2,3,4,6-tetra-O-benzylglucopyranosyl)-5H-indolo[2,3-a]pyrrolo[3,4-c]carbazole--5,7(6H)-dione.

### Example 31

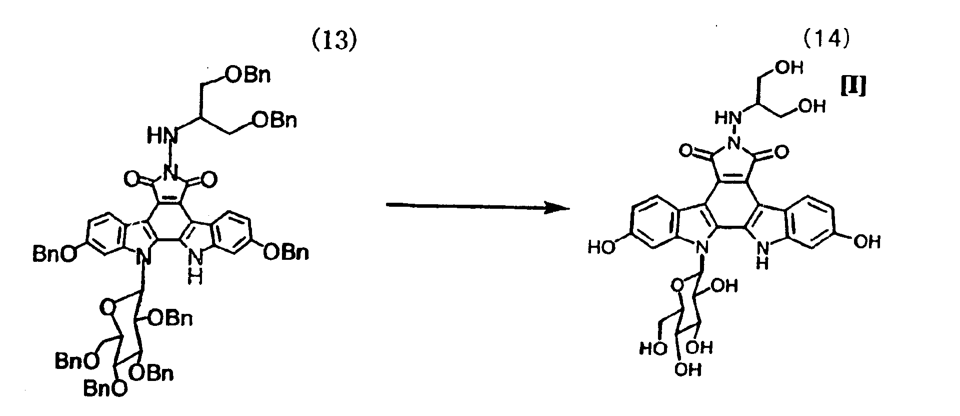

10 % Palladium/carbon (50 w/w %, 112 g) was placed in a hydrogenation vessel, and a solution of 12-β-D-(2,3,4,6-tetra-O-benzylglucopyranosyl)-12,13-dihydro-2,10-dibenzyloxy-6-[[(2-benzyloxy-1-(benzyloxymethyl)ethyl)amino]-5H-indolo[2,3-a]pyrrolo[3,4-c]carbazole-5,7(6H)-dione (13) in tetrahydrofuran (175 g/L, 6.4 L, 1.12 kg), isopropanol (7.9 L) and 3N HCl (224 mL) were added thereto. The mixture was hydrogenated while vigorous stirring at 40°C under a hydrogen pressure of 40 psi for 4 to 14 hours until absorbance of hydrogen becomes to be theoretically 110 %. The reaction solution was cooled to 25°C and filtered with Solka Floc (Registered Trade Mark) to collect solids such as catalyst. Such solid was washed with isopropanol/tetrahydrofuran (3:2) (3L x 1). The filtrate and the washing were combined, and the solution was adjusted to pH 2.5 with 1M triethylamine/isopropanol (about 600 mL). After addition of water (4.0 L), the solution was concentrated to a volume of 7.5 L in atmospheric pressure, further concentrated while adding isopropanol/water (4:1) (6.5 L), and finally concentrated to a water content of 20 % (w/v) while further supplying isopropanol (about 9 L) and maintaining the volume at about 7.5 L. The concentrate was kept at 70°C, and a suspension of seed crystals (5 g) in isopropanol (50 mL) was added thereto. The mixture was kept at 70°C for one hour, and isopropanol (5.0 L) was added over a period of 1.5 hours. The resultant solution was kept at 70°C for 9 to 24 hours to precipitate crystals. The resultant suspension was concentrated in atmospheric pressure while supplying isopropanol (17 L) until water content in the concentrate becomes to be 3 w/v %. The suspension was kept at 70°C for 3 to 6 hours, cooling to 22°C and kept at the same temperature for one hour. The suspension was filtered to give a cake, which was washed successively with isopropanol (2.5 L) and methanol (1.5 L). The cake was dried in vacuo at 38°C for 6 hours to give orange crystals in 80 % or higher yield (content: 99 % or higher). Since mass spectrum, NMR spectrum and IR spectrum of the above orange crystals agreed to those of the compound of Example 6 described in WO 95/30682, the objective compound (14) of this Example was identified as 12,13-dihydro-2,10-dihydroxy-6-N-(1-hydroxymethyl-2-hydroxyethylamino)-13-(β-D-glucopyranosyl)-5H-indolo[2,3-a]pyrrolo[3,4-c]carbazole-5,7(6H)-dione.
Measurement conditions of high performance liquid chromatography
Separation column: YMC ODS-AQ (250 x 4.6 mm)
Flow rate: 1.5 mL/min
Detection wave length: 228 nm
Mobile phase: A=0.1 % H₃PO₄-water, B=acetonitrile
Injection amount: 10 µL
Measurement temperature: 25°C

### Example 32

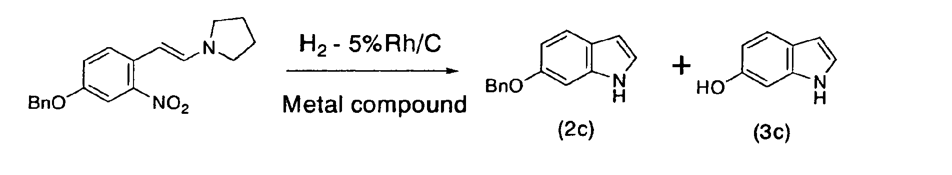

3-Benzyloxy-6-(2-pyrrolidinylvinyl)nitrobenzene(1.00 g, 3.08 mL), 5 % rhodium/carbon powder (63.5 mg, 0.0309 mmol), ferrous(II) acetate (5.6 mg, 0.0309 mmol) as a metal compound, and tetrahydrofuran (20 mL) were placed in a 30 mL eggplant type flask equipped with a magnetic stirrer under nitrogen atmosphere and then the nitrogen gas was substituted with hydrogen gas. The resultant suspension was stirred at room temperature for 41 hours under hydrogen atmosphere, and then the hydrogen gas was substituted with nitrogen gas, and stirred overnight under nitrogen atmosphere. The reaction mixture was filtered to obtain a solid, which was washed with tetrahydrofuran. The filtrate and the washing were combined to give a brown solution (79.83 g). The solution was analyzed by high performance liquid chromatography, indicating that the objective 6-benzyloxyindole(2c)(661 mg, 96 % yield) and by-produced 6-hydroxyindole (3c)(2 mg, 0.6 % yield) were obtained.
6-Benzyloxyindole:
¹H-NMR (500MHz, DMSO-d₆, δppm): 10.90 (br.s, 1H), 7.49 (d, J= 7.5 Hz, 1H), 7.44 (d, J= 8.6 Hz, 1H), 7.40 (dd, J= 7.5, 7.5 Hz, 1H), 7.33 (dd, J= 7.5, 7.5 Hz, 1H), 7.21 (br.dd, J= 2.4, 2.4 Hz, 1H), 7.01 (br. m, 1H), 6.77 (dd, J= 1.8, 8.6 Hz, 1H), 6.36 (br. m, 1H), 5.12 (s, 2H).
¹³C-NMR (126MHz, DMSO-d₆, δppm): 154.7, 138.0, 136.8, 128.7, 128.0, 127.9, 124.4, 122.5, 120.9, 110.1, 101.3, 96.3, 69.9
6-Hydroxyindole:
¹H-NMR (500MHz, DMSO-d₆, δppm): 10.68 (br.s, 1H), 8.88 (br.s, 1H), 7.33 (d, J= 8.4 Hz, 1H), 7.12 (m,1H), 6.79 (m,1H), 6.56 (dd, J= 8.4, 1.5 Hz, 1H), 6.30 (m,1H).
¹³C-NMR (126MHz, DMSO-d₆, δppm): 153.78, 137.90, 124.00, 121.90, 121.17, 110.37, 101.75, 97.36.

### Examples 33 to 35

Following a procedure similar to Example 32, 2-(2-pyrolidinylvinyl)nitrobenzene was hydrogenated in the presence of 5 % rhodium/carbon powder, and as a metal compound, ferrous(II) acetate, nickel(II) nitrate or cobalt(III) acetylacetonate as the catalyst of the present invention. Indole was obtained in high yield as shown in Table 3 below.

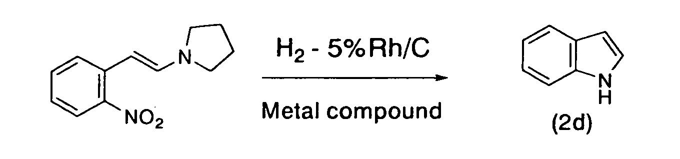

**Table 3**

| Example No. | 5 % RH/C (Amount used) | Metal compound (Amount used) | Reaction time | Yield (%) |
|---|---|---|---|---|
| 33 | 1 mol % | Fe(OAc)₂ (1 mol %) | 31 hours | 88 |
| 34 | 3 mol % | Ni(NO₃)₂·6H₂O (20 mol %) | 32 hours | 82 |
| 35 | 1 mol % | Co(acac)₃ (5 mol %) | 38 hours | 85 |

### Indole:

The product (2d) was identified as a result of comparison with various spectrum of commercially available products.

### Example 36 to 38

Following a procedure similar to Example 32, 3-benzyloxy-2-(2-pyrrolidinylvinyl)nitrobenzene as a starting material was reduced in the presence of 5 % rhodium/carbon powder and as a metal compound, ferrous(II) acetate, nickel(II) nitrate or cobalt (III) acetylacetonate as the catalyst of the present invention.

The results are shown in the following Table 4, and in the case where the reducing agent of the present invention was used, 4-benzyloxyindole was obtained in a higher yield and 4-hydroxyindole was byproduced in a lower yield, compared to Comparison Example 3.

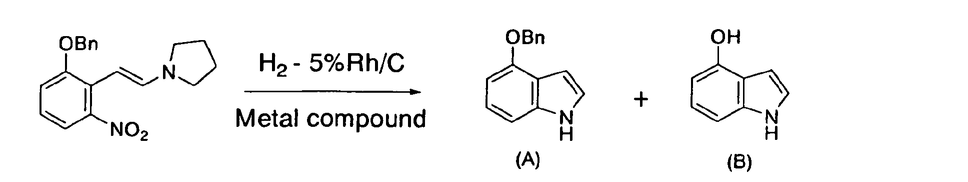

**Table 4**

| Example No. | 5 % RH/C Amount used | Metal compound | Reaction time | Compound (A) Yield (%) | Compound (B) Yield (%) |
|---|---|---|---|---|---|
| 36 | 1 mol % | Fe(OAc)₂ (1 mol %) | 31 hours | 83 | 5 |
| 37 | 3 mol % | Ni(NO₃)₂·6H₂O (20 mol%) | 32 hours | 88 | 6 |
| 38 | 1 mol % | Co(acac)₃ (5 mol %) | 38 hours | 80 | 3 |
| Comparison Example 3 | 3 mol % | None | 167 hours | 43 | 38 |

4-Benzyloxyindole:
¹H-NMR (500MHz, CDCl₃, δppm): 8.12 (br.s, 1H), 7.49 (br.d, J= 7.5 Hz, 1H), 7.44 (br.t, J= 7.5 Hz, 1H), 7.37 (m, 1H), 7.14 (dd, J= 8.0, 7.8 Hz, 1H), 7.10 (m, 1H), 7.04 (d, J= 8.0 Hz, 1H), 6.77 (m, 1H), 6.64 (d, J= 7.7 Hz, 1H), 5.28 (s, 2H).
¹³C-NMR (126MHz, CDCl₃, δppm): 152.88,137.94,137.66,128.81, 128.05, 127.66,123.00,119.23,105.02,101.46,100.40,70.27.
4-Hydroxyindole:
¹H-NMR (500MHz, CDCl₃, δppm): 8.19 (br.s, 1H), 7.13 (m, 1H), 7.06 (dd,J= 8.0, 7.6 Hz, 1H), 7.01 (d,J= 8.0 Hz, 1H), 6.62 (m, 1H), 6.54 (d,J= 7.6 Hz, 1H), 5.22 (br.s, 1H).
¹³C-NMR (126MHz, CDCl₃, δppm): 149.08, 137.86, 123.18, 123.03, 117.64, 104.37, 104.29, 98.91.

### Example 39 to 41

Following a procedure similar to Example 32, 4-benzyloxy-2-(2-pyrrolidinylvinyl)nitrobenzene as a starting material was reduced in the presence of 5 % rhodium/carbon powder and as a metal compound, ferrous(II) acetate, nickel(II) nitrate or cobalt(III) acetylacetonate as the catalyst of the present invention.

The results are shown in the following Table 5, and in the case where the reducing agent of the present invention was used, 5-benzyloxyindole was obtained in a higher yield and 5-hydroxyindole was byproduced in a lower yield, compared to Comparison Example 4.

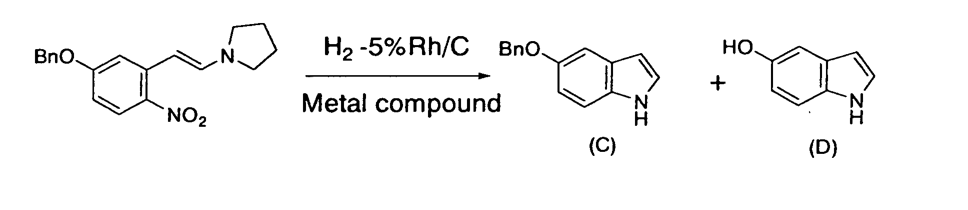

**Table 5**

| Example No. | 5 % RH/C Amount used (mol %) | Metal compound (Amount used) | Reaction time | Compound (C) Yield (%) | Compound (D) Yield (%) |
|---|---|---|---|---|---|
| 39 | 1 mol % | Fe(OAc)₂ (1 mol %) | 9 hours | 99 | 1 |
| 40 | 3 mol % | Ni(NO₃)₂·6H₂O (20 mol %) | 23 hours | 93 | 0.4 |
| 41 | 1 mol % | Co(acac)₃ (5 mol %) | 15 hours | 96 | 3 |
| Comparison Example 4 | 3 mol % | None | 15 hours | 86 | 14 |

5-Benzyloxyindole:
¹H-NMR (500MHz, CDCl₃, δppm): 7.92 (br.s, 1H), 7.44 (br.d, J= 7.5 Hz, 1H), 7.36-7.26 (m, 2H), 7.17-7.15 (m, 2H), 7.03 (m, 1H), 6.92 (dd, J= 8.8, 2.4 Hz, 1H), 6.42 (m, 1H), 5.06 (s, 2H).
¹³C-NMR (126MHz, CDCl₃, δppm): 153.64, 137.99,131.45,128.82, 128.53,128.08,127.89,125.30,113.29,112.06, 104.32, 102.59, 71.26.
5-Hydroxyindole:
¹H-NMR (500MHz, DMSO-d₆, δppm): 10.76 (br.s, 1H), 8.61 (br.s, 1H), 7.24 (m, 1H), 7.21 (d,J= 8.6 Hz, 1H), 6.89 (br.d,J= 2.0 Hz, 1H), 6.65 (dd, J= 8.6, 2.0 Hz, 1H), 6.27 (m, 1H).
¹³C-NMR (126MHz, DMSO-d₆, δppm): 151.42, 131.38, 129.30, 126.37, 112.50, 112.20, 104.76, 101.12.

### Examples 42 to 44

Following a procedure similar to Example 32, 5-benzyloxy-2-(2-pyrrolidinylvinyl)nitrobenzene as a starting material was reduced in the presence of 5 % rhodium/carbon powder and as a metal compound, ferrous(II) acetate, nickel(II) nitrate or cobalt(III) acetylacetonate as the catalyst of the present invention.

The results are shown in the following Table 6, and in the case where the reducing agent of the present invention was used, 6-benzyloxyindole was obtained in a higher yield and 6-hydroxyindole was byproduced in a lower yield, compared to Comparison Example 5.

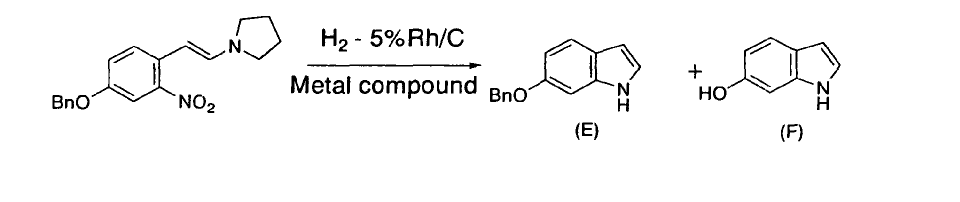

**Table 6**

| Example No. | 5 % RH/C Amount used | Metal compound (Amount used) | Reaction temperature | Compound (E) Yield (%) | Compound (F) Yield (%) |
|---|---|---|---|---|---|
| 42 | 1 mol % | Fe(OAc)₂ (1 mol %) | 9 hours | 96 | 0.6 |
| 43 | 3 mol % | Ni(NO3)₂·6H₂O (20 mol %) | 23 hours | 90 | 1 |
| 44 | 1 mol % | Co(acac)₃ (5 mol %) | 15 hours | 94 | 3 |
| Comparison Example 5 | 3 mol % | None | 42 hours | 55 | 34 |

### Example 45

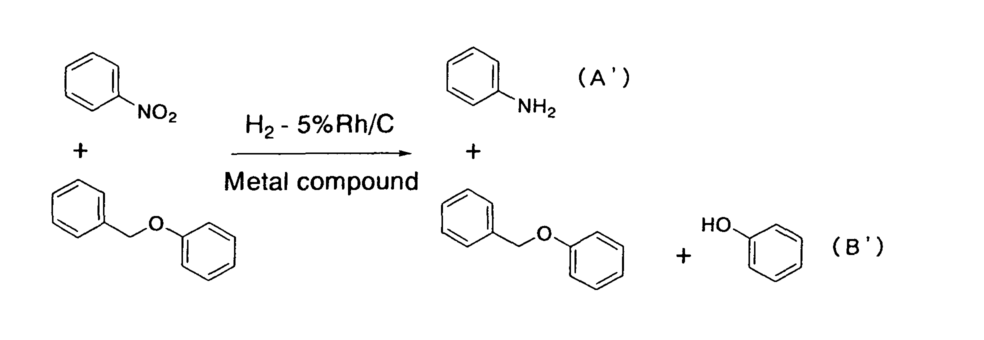

Nitrobenzene (379 mg, 3.08 mmol), benzylphenyl ether (567 mg, 3.08 mmol) as a substrate, 5 % rhodium/carbon powder (63.5 mg, 0.0309 mmol), ferrous(II) acetate (5.6 mg, 0.0309 mmol) as a metal compound and tetrahydrofuran (20 mL) were placed in a 30 mL eggplant type flask equipped with a magnetic stirrer under nitrogen atmosphere. After the nitrogen gas was substituted with hydrogen gas, the resultant suspension was stirred at room temperature for 16 hours under hydrogen atmosphere, and after the hydrogen gas was substituted with nitrogen gas, the resultant suspension was stirred overnight under nitrogen atmosphere. After removal of the residual solid by filtration, the residue was washed with tetrahydrofuran. The filtrate and the washing were combined to give a brown solution. The solution was analyzed by high performance liquid chromatography, indicating that aniline (A'), benzylphenyl ether as the recovered substrate and phenol (B') as a reduced substrate were obtained in 86 % yield (crop 247 mg), 89 % recovery (crop 503 mg) and 1 % yield (crop 4 mg), respectively.

Each component in the resultant solution was identified as a result of comparison with various spectrum of commercially available products.

### Examples 46 to 48

Following a procedure similar to Example 45, reduction reaction was carried out using 5 % rhodium/carbon powder, ferrous(II) acetate, nickel(II) nitrate or cobalt(III) acetylacetonate as a metal compound as the catalyst of the present invention, and benzylphenyl ether as a substrate. The results are shown in the following Table 7.

Aniline and the reduced substrate (phenol) were identified as a result of comparison with various spectrum of commercially available products.

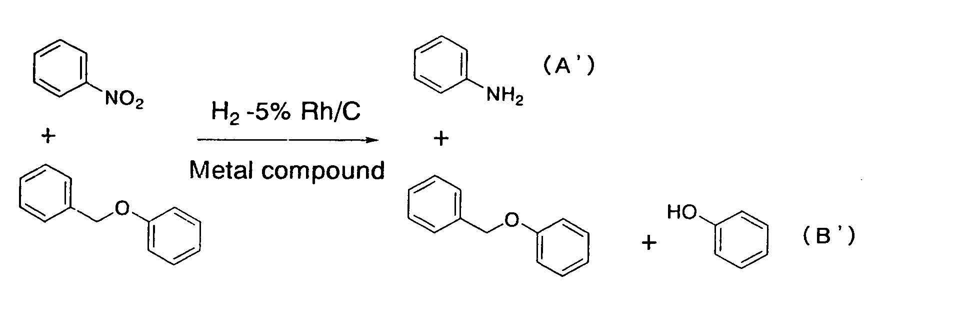

**Table 7**

| Example No. | 5%Rh/C Amount used | Metal compound (Amount used) | Reaction time | Yield (A') | Yield (B') |
|---|---|---|---|---|---|
| 46 | 3 mol% | Ni(NO₃)₂·6H₂O (20 mol %) | 16 hours | 86 % | 32 % |
| 47 | 1 mol% | Fe(OAc)₂ (1 mol %) | 16 hours | 86 % | 1 % |
| 48 | 1 mol% | Co(acac)₃ (5 mol %) | 16 hours | 85 % | 1 % |
| Comparison Example 6 | 3 mol% | None | 16 hours | 64 % | 27 % |
| Comparison Example 7 | 1 mol% | None | 16 hours | 62 % | 15 % |
| Yield (A' ) is a yield of aniline, and yield (B' ) is a yield of phenol. | | | | | |

### Examples 49 to 51

Following a procedure similar to Example 45, reduction reaction was carried out using 5 % rhodium/carbon powder, ferrous(II) acetate, nickel(II) nitrate or cobalt(III) acetylacetonate as a metal compound as the catalyst of the present invention, and chlorobenzene as a substrate. The results are shown in Table 8 below.

Aniline and the reduced substrate (benzene) were identified as a result of comparison with various spectrum of commercially available products.

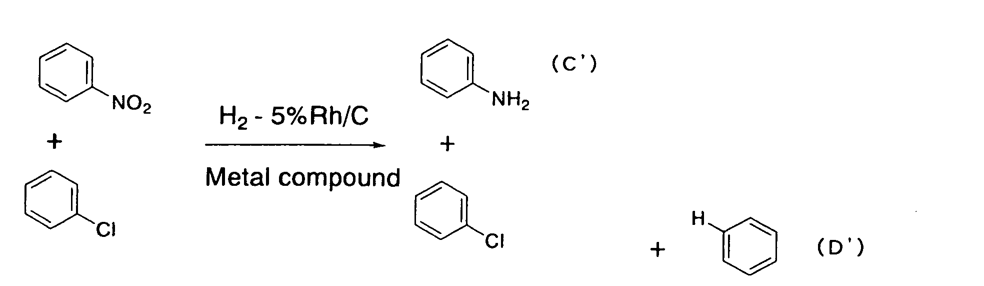

**Table 8**

| Example No. | 5 % Rh/C Amount used | Metal compound (Amount used) | Reaction time | Yield (C') | Yield (D') |
|---|---|---|---|---|---|
| 49 | 3 mol % | Ni(NO₃)₂·6H₂O (20 mol %) | 16 hours | 91 % | 24 % |
| 50 | 1 mol % | Fe(OAc)₂ (1 mol %) | 16 hours | 9 0 % | 4 % |
| 51 | 1 mol % | Co(acac)₃ (5 mol %) | 16 hours | 94 % | 3 % |
| Comparison Example 8 | 3 mol % | None | 16 hours | 10 % | 78 % |
| Comparison Example 9 | 1 mol % | None | 16 hours | 65 % | 33 % |
| Yield (C' ) is a yield of aniline, and yield (D' ) is a yield of benzene. | | | | | |

### Examples 52 to 54

Following a procedure similar to Example 45, reduction reaction was carried out using 5 % rhodium/carbon powder, ferrous(II) acetate, nickel(II) nitrate or cobalt(III) acetylacetonate as a metal compound as the catalyst of the present invention, and benzaldehyde as a substrate. The results are shown in Table 9 below.

Aniline and the reduced substrate (benzyl alcohol) were identified as a result of comparison with various spectrum of commercially available products.

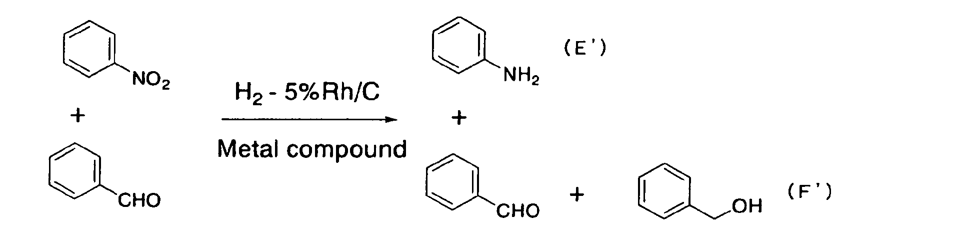

**Table 9**

| Example No. | 5 % Rh/C Amount used | Metal compound (Amount used) | Reaction time | Yield (E' ) | Yield (F' ) |
|---|---|---|---|---|---|
| 52 | 3 mol % | Ni(NO₃)₂·6H₂O (20 mol %) | 16 hours | 83% | 16% |
| 53 | 1 mol % | Fe(OAc)₂ (1 mol %) | 16 hours | 94% | 7% |
| 54 | 1 mol % | Co(acac)₃ (5 mol %) | 16 hours | 81% | 8% |
| Comparison Example 10 | 3 mol % | None | 16 hours | 65% | 56% |
| Comparison Example 11 | 1 mol % | None | 16 hours | 92% | 29% |
| Yield (E') is a yield of aniline and yield (F') is a yield of benzyl alcohol. | | | | | |

### Example 55

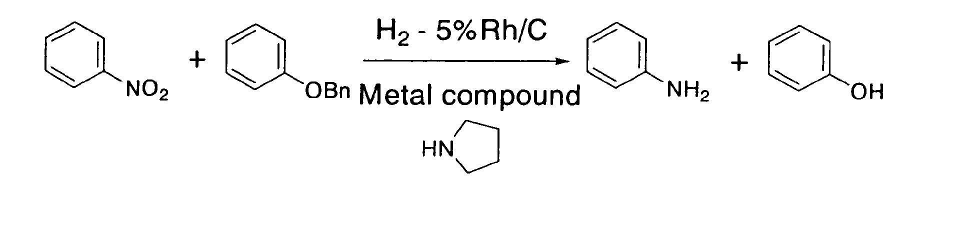

Nitrobenzene (379 mg, 3.08 mmol), benzyl phenyl ether (567 mg, 3.08 mmol), pyrrolidine (0.257 mL, 3.08 mmol), 5 % rhodium/carbon powder (63.5 mg, 0.0309 mmol), ferrous(II) acetate (5.6 mg, 0.0309 mmol) as a metal compound and tetrahydrofuran (20 mL) were placed in a 30 mL eggplant type flask equipped with a magnetic stirrer under nitrogen atmosphere. After the nitrogen gas was substituted with hydrogen gas, the resultant suspension was stirred at room temperature for 5 hours under hydrogen atmosphere, and stirred overnight under nitrogen atmosphere. After removal of the residual solid by filtration, the residue was washed with tetrahydrofuran. The filtrate and the washing were combined to give a brown solution. The solution was analyzed by high performance liquid chromatography, indicating that aniline was obtained in 88 % yield (crop 252 mg,) and phenol derived from reduction of benzylphenyl ether was by-produced in 0.4 % yield (crop 1 mg).

### Examples 56 to 58

Following a procedure similar to Example 55, reduction reaction was carried out using 5 % rhodium/carbon powder; ferrous(II) acetate, nickel(II) nitrate or cobalt(III) acetylacetonate as a metal compound; and pyrrolidine as a base as the catalyst of the present invention. The results are shown in Table 10 below.

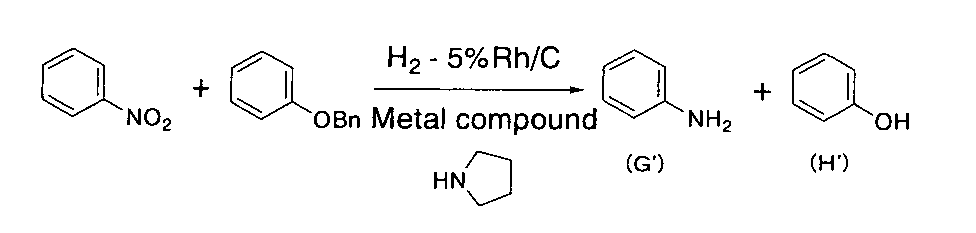

**Table 10**

| Example No. | 5 % Rh/C Amount used | Metal compound (Amount used) | Reaction time | Yield (G'): % | Yield (H'): % |
|---|---|---|---|---|---|
| 56 | 3 mol % | Ni(NO₃)₂·6H₂O (20mol%) Pyrrolidine (1 eq.) | 5 hours | 90 | 8 |
| 57 | 1 mol % | Fe(OAc)₂ (1mol%) Pyrrolidine (1 eq.) | 5 hours | 88 | 0.4 |
| 58 | 1 mol % | Co(acac)₃ (5mol%) Pyrrolidine (1 eq.) | 5 hours | 94 | 6 |
| Comparison Example 12 | 3 mol % | No use of metal compound Pyrrolidine (1 eq.) | 5 hours | 8 6 | 42 |
| Comparison Example 13 | 3 mol % | No use of metal compound pyrrolidine (1 eq.) | 2 hours | 92 | 20 |
| Comparison Example 14 | 1 mol % | No use of metal compound Pyrrolidine (1 eq.) | 5 hours | 91 | 14 |
| Yield (G') is a yield of aniline and yield (H') is a yield of phenol. The abbreviation "eq." means equivalent. | | | | | |

It was confirmed from the above results that selectivity of the functional groups to be reduced is improved by further addition of an amine in the catalytic reduction in the presence of a catalyst of the present invention.

### Example 59

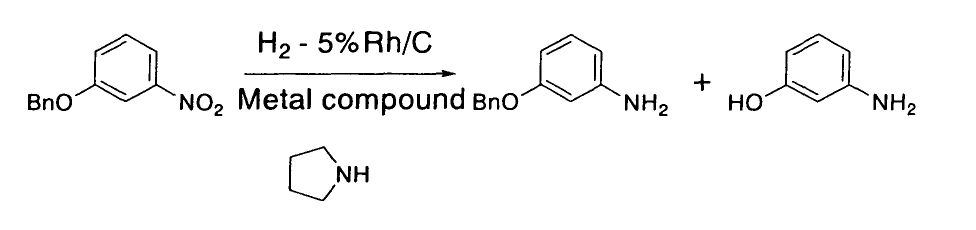

3-Benzyloxynitrobenzene (706 mg, 3.08 mmol), pyrrolidine (0.257 mL, 3.08 mmol), 5% rhodium/carbon powder (190 mg, 0.0924 mmol), nickel(II) nitrate hexahydrate (179 mg, 0.616 mmol) and tetrahydrofuran (20 mL) were placed in a 30 mL eggplant type flask equipped with a magnetic stirrer under nitrogen atmosphere. After the nitrogen gas was substituted with hydrogen gas, the resultant suspension was stirred at room temperature for 2.5 hours under hydrogen atmosphere, and the reaction system was substituted for nitrogen atmosphere. The resultant solid was collected by filtration and washed with tetrahydrofuran. The filtrate and the washing were combined to give a brown solution. The solution was analyzed by high performance liquid chromatography, indicating that 3-benzyloxyaniline was obtained in 92 % yield (crop 565 mg) and 3-hydroxyaniline in 1 % yield (crop 3 mg).

### Examples 60 to 66

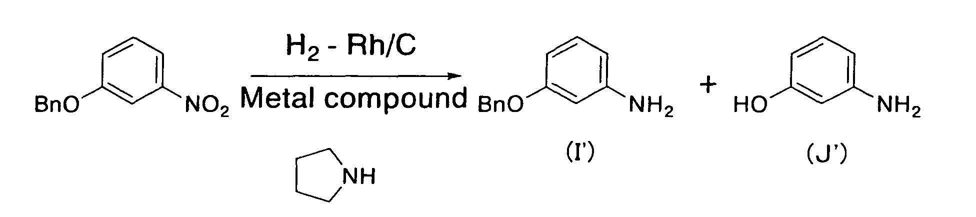

Following a procedure similar to Example 59, reduction reaction was carried out using ferrous(II) acetate, nickel(II) nitrate or cobalt(III) acetylacetonate as a metal compound. The results are shown in Table 11 below.

**Table 11**

| Example No. | 5% Rh/C Amount used | Metal compound Amount used | pyrrolidine | Reaction time | Yield (I') | Yield (J') |
|---|---|---|---|---|---|---|
| 60 | 3 mol% | Ni(NO₃)₂·6H₂O (20 mol%) | None | 15 hours | 92 | 2 |
| 61 | 3 mol% | Ni(NO₃)₂·6H₂O (20 mol%) | 1 eq. | 2.5 hours | 92 | 1 |
| 62 | 3 mol% | Ni(NO₃)₂·6H₂O (20 mol%) | 3 eq. | 2.5 hours | 95 | 0.5 |
| 63 | 1 mol% | Fe(OAc)₂ (1 mol%) | None | 33 hours | 92 | 0.8 |
| 64 | 1 mol% | Fe(OAc)₂ (1 mol%) | 1 eq. | 2.5 hours | 98 | 0.5 |
| 65 | 1 mol% | Co(acac)₃ (5 mol%) | None | 33 hours | 89 | 0.6 |
| 66 | 1 mol% | Co(acac)₃ (5 mol%) | 1 eq. | 4 hours | 91 | 7 |
| Comparison Example 15 | 3 mol% | None | None | 15 hours | 76 | 11 |
| Comparison Example 16 | 3 mol% | None | 1 eq. | 2.5 hours | 79 | 15 |
| Comparison Example 17 | 3 mol% | None | 3 eq. | 2.5 hours | 86 | 14 |
| Yield (I'): yield (%) of 3-benzyloxyaniline | | | | | | |
| Yield (J'): yield (%) of 3-hydroxyaniline | | | | | | |

It was confirmed from the results of Examples 60 to 66 that when an amine such as pyrrolidine was added in catalytic reduction system of the present invention using rhodium/carrier catalyst and using a metal compound such as iron salts, nickel salts or cobalt salts, the reaction rate was dramatically improved, the probability of reduction of functional groups such as benzyl ether which was liable to be reduced was lowered, nitro group was selectively reduced and the yield was increased.

### Examples 67 to 68

Following a procedure similar to Example 45, reduction reaction was carried out using 5 % rhodium/carbon powder, and ferrous(II) acetate, nickel(II) nitrate or cobalt(III) acetylacetonate as a metal compound as a catalyst of the present invention, and styrene (K' ) as a substrate. The results are shown in the following table.

Aniline and the reduced substrate (ethylbenzene (L')) were identified as a result of comparison with various spectrum of commercially available products.

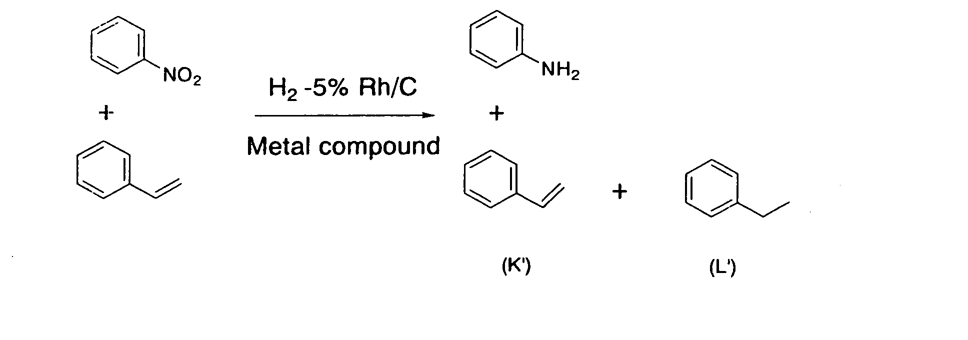

**Table 12**

| Example No. | 5 % Rh/C Amount used | Metal compound (Amount used) | Reaction time | Yield (K'): % | Yield (L'): % |
|---|---|---|---|---|---|
| 68 | 1 mol % | Fe(OAc)₂ (1 mol %) | 16 hours | 80% | 98% |
| 69 | 1 mol % | Co(acac)₃ (5 mol %) | 16 hours | 89% | 99% |
| Comparison Example 18 | 3 mol % | None | 16 hours | 31% | 93% |
| Comparison Example 19 | 1 mol % | None | 16 hours | 73% | 98% |
| Yield (K'): yield of aniline | | | | | |
| Yield (L'): yield of ethylbenzene | | | | | |

It was confirmed from the above results that when catalytic reduction was carried out using the catalyst of the present invention, nitro group and vinyl group were reduced without reduction of benzyl ether, chloro and aldehyde groups.

Therefore, when catalytic reduction is carried out using the catalyst of the invention, i.e. a catalyst comprising a rhodium-carrier catalyst and iron salts, nickel salts or cobalt salts as a metal compound, nitro group, alkenyl group or alkynyl group can be selectively reduced without reduction of functional groups such as benzyl ether, aryl halides, aldehydes, ketones, etc.

### Industrial Applicability

The objective compound (I) of the present invention is useful as a medicine, and the present invention relates to an industrially advantageous process for producing the said compound. Additionally, the catalyst of the present invention can be utilized in various reduction reactions as a catalyst by which selective reduction is made available.

## Claims

1. A process for producing an indolopyrrolocarbazole derivative represented by the formula (I), which comprises the following steps:
(i): the step of reacting a compound of the formula (XIII) 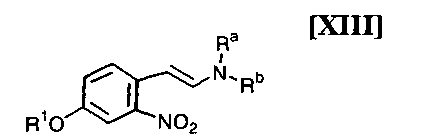 wherein R¹ represents a hydroxy protecting group, and R^{a} and R^{b} each independently represents a C₁-C₇ alkyl group, or R^{a} and R^{b} may be combined together to form a C₃-C₆ alkylenyl group, or a salt thereof with hydrogen gas in the presence of a rhodium compound and a metal compound to produce an indole compound of the formula (XII): 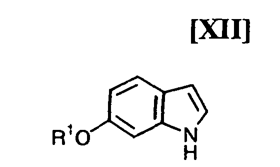 wherein R¹ has the same meaning as defined above, or a salt thereof;
(ii): the step of reacting the resulting indole compound of the formula (XII) or a salt thereof with a magnesium chloride of the formula (XI):
R^{c}MgCl [XI]
wherein R^{c} represents a C₁-C₇ alkyl group, a phenyl group, a vinyl group or an allyl group; or a magnesium compound of the formula (X):
R^{d}MgR^{d} [X]
wherein R^{d} represents a C₁-C₇ alkyl group or a phenyl group, or a salt thereof, followed by reacting the resulting product with a maleimide compound of the formula (IX): 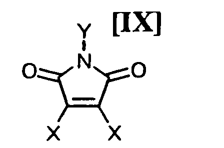 wherein X represents a halogen atom, and Y represents a hydrogen atom, a C₁-C₇ alkyl group, a phenyl group, a benzyloxymethyl group, or a C₇-C₁₂ aralkyl group, to produce a bis-indole compound of the formula (VIII): 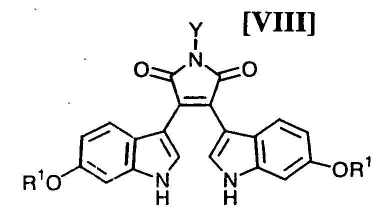 wherein R¹ and Y have each the same meaning as defined above, or a salt thereof;
(iii): the step of subjecting the resulting bis-indole compound (VIII) or a salt thereof to ring-closure reaction to produce a compound of the formula (VII): 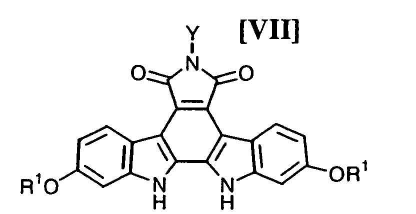 wherein R¹ and Y have each the same meaning as defined above, or a salt thereof;
(iv): the step of coupling the resulting compound (VII) or a salt thereof with an activated glucose derivative of the formula (VI): 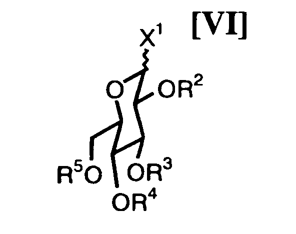 wherein each R², R³, R⁴ and R⁵ is a hydroxy protecting group, and X¹ represents a halogen atom, to produce a compound of the formula (V): 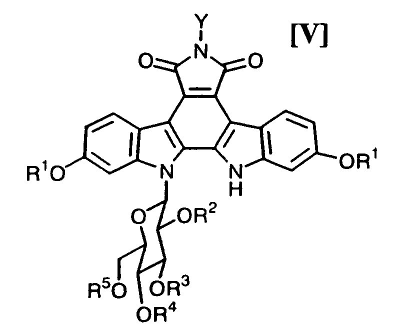 wherein R¹, R², R³, R⁴, R⁵ and Y have each the same meaning as defined above, or a salt thereof;
(v): the step of treating the resulting compound (V) or a salt thereof with a base to produce a compound of the formula (IV): 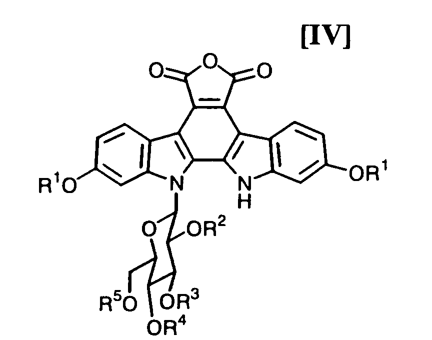 wherein R¹, R², R³, R⁴ and R⁵ have each the same meaning as defined above, or a salt thereof;
(vi): the step of reacting compound (IV) or a salt thereof with a compound of the formula (III): 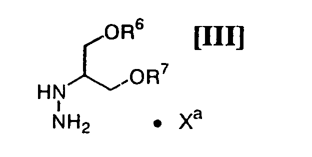 wherein R⁶ and R⁷ each represents a hydroxy protecting group, and X^{a} represents an acid molecule to produce a compound of the formula (II): 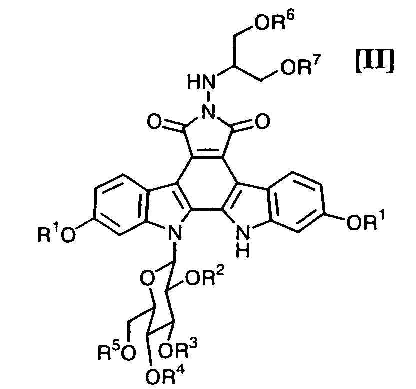 wherein R¹, R², R³, R⁴, R⁵, R⁶ and R⁷ have each the same meaning as defined above, or a salt thereof; and
(vii): the step of deprotecting the resulting compound (II) or a salt thereof to produce an indolopyrrolocarbazole derivative of the formula (I): 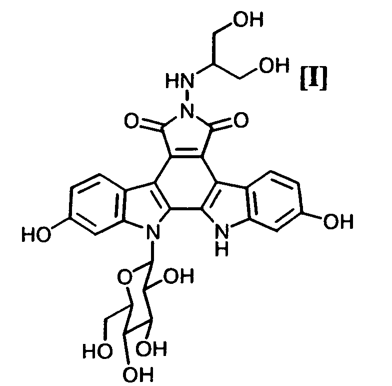 or a salt thereof.

2. The process according to Claim 1, wherein the rhodium compound is rhodium-carbon, rhodium-alumina, rhodium-calcium carbonate or rhodium-barium sulfate.

3. The process according to Claim 1, wherein the metal compound is a nickel(II) compound, an iron(II) compound, an iron(III) compound, a cobalt(II) compound or a cobalt(III) compound.

4. The process according to Claim 3, wherein the nickel(II) compound, the iron(II) compound, the iron(III) compound, the cobalt(II) compound or the cobalt(III) compound are NiBr₂, Ni(NO₃)₂, Ni(OCOCH₃)₂, FeBr₃, FeCl₂, FeSO₄ , FeCl₃, FeCl₃-SiO₂, Fe(OCOCH₃)₂, Fe(II)fumarate, CoBr₂, CoCl₂, 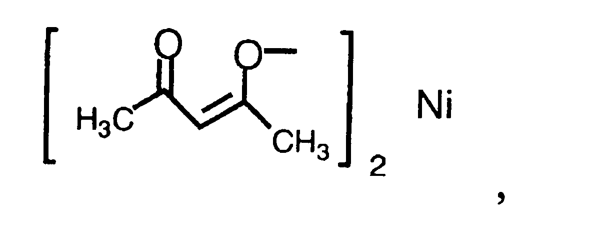 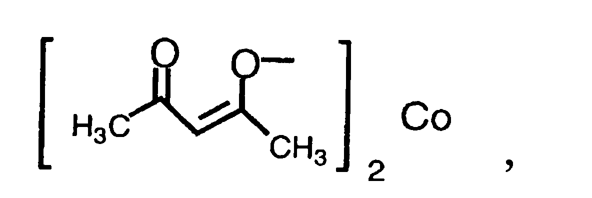 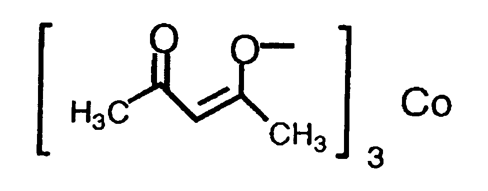 or

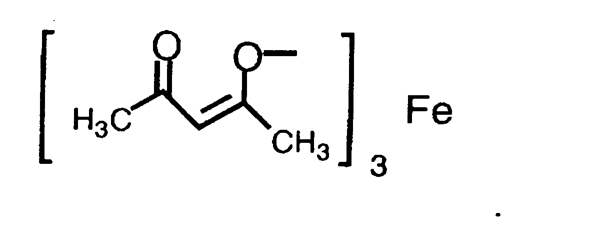

5. The process according to Claim 1, wherein R¹, R², R³, R⁴, R⁵, R⁶ and R⁷ each represents a benzyl group.

6. The process according to Claim 1, wherein the magnesium chloride of the formula (XI) is ethyl magnesium chloride, isopropyl magnesium chloride or n-butyl magnesium chloride.

7. The process according to Claim 1, wherein the magnesium compound of the formula (X) is di(n-butyl)magnesium, di(s-butyl)magnesium, (n-butyl)(s-butyl)magnesium, dimethyl magnesium or diethyl magnesium.

8. The process according to Claim 1, wherein the maleimide compound of the formula (IX) is a maleimide compound represented by the formula (IX-a): 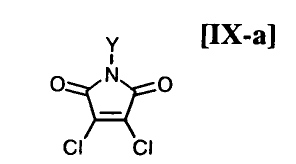 wherein Y represents a hydrogen atom, a C₁-C₇ alkyl group, a phenyl group, a benzyloxymethyl group or an aralkyl group.

9. The process according to Claim 1, wherein Y is a methyl group.

10. The process according to Claim 1, wherein X^{a} is oxalic acid.

11. The process according to Claim 1, wherein the coupling is conducted in the presence of a phase transfer catalyst.

12. A process for producing an indole compound or a salt thereof, which comprises producing an indole compound represented by the formula (XII): 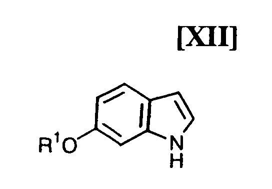 wherein R¹ is a hydroxy protecting group, or a salt thereof by reacting a compound represented by the formula (XIII): 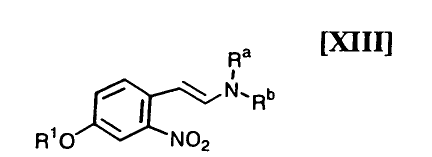 wherein R¹ has the same meaning as defined above, and R^{a} and R^{b} each independently represents a C₁-C₇ alkyl group, or R^{a} and R^{b} may be combined together to form a C₃-C₆ alkylenyl group, with hydrogen gas in the presence of a rhodium compound and a metal compound.

13. The process according to Claim 13, which comprises reacting a compound represented by the formula (XIII): 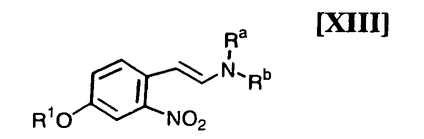 wherein R¹ is a hydroxy protecting group, and R^{a} and R^{b} each independently represents a C₁-C₇ alkyl group, or R^{a} and R^{b} may be combined together to form a C₃-C₆ alkylenyl group, or a salt thereof with hydrogen gas in the presence of a rhodium compound and a metal compound, and treating the resulting crude product with silica gel.

14. A process for producing a bis-indole compound or a salt thereof, which comprises reacting an indole compound of the formula (XII): 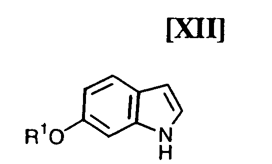 wherein R¹ represents a hydroxy protecting group, or a salt thereof with a magnesium chloride of the formula (XI):
R^{c}MgCl [XI]
wherein R^{c} represents a C₁-C₇ alkyl group, a phenyl group, a vinyl group or an allyl group; or a magnesium compound of the formula (X):
R^{d}MgR^{d} [X]
wherein R^{d} represents a C₁-C₇ alkyl group or a phenyl group, or a salt thereof, or a mixture of the magnesium chloride of the formula (XI) and the magnesium compound of the formula (X) in an inert solvent, followed by reacting the resulting product with a maleimide compound of the formula (IX): 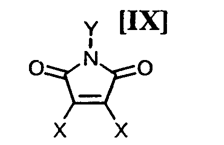 wherein X represents a halogen atom; and Y represents a hydrogen atom, a C₁-C₇ alkyl group, a phenyl group, a benzyloxymethyl group or a C₇-C₁₂ aralkyl group, preferably in an inert solvent to produce a bis-indole compound of the formula (VIII): 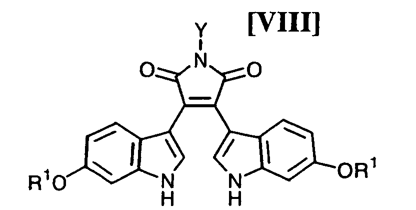 wherein R¹ and Y have each the same meaning as defined above, or a salt thereof.

15. The process according to Claim 14, wherein the maleimide compound of the formula (IX) is a maleimide compound represented by the formula (IX-a): 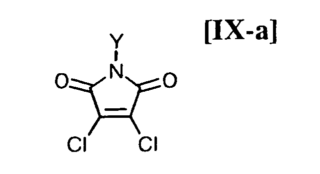 wherein Y represents a hydrogen atom, a C₁-C₇ alkyl group, a phenyl group, a benzyloxymethyl group or a C₇-C₁₂ aralkyl group.

16. A process for producing a compound represented by the formula (VII): 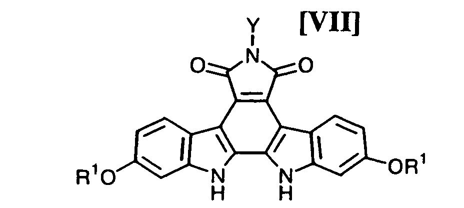 wherein R¹ represents a hydroxy protecting group, and Y represents a hydrogen atom, a C₁-C₇ alkyl group, a phenyl group, a benzyloxymethyl group or a C₇-C₁₂ aralkyl group, or a salt thereof, which comprises treating a bis-indole compound represented by the formula (VIII): 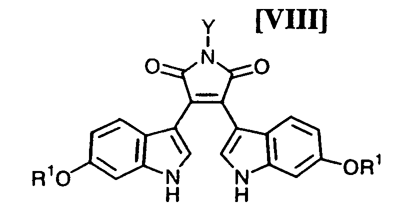 wherein R¹ and Y have each the same meaning as defined above, or a salt thereof with 2,3-dichloro-5,6-dicyano-1,4-benzoquinone in a nonpolar solvent for ring-closure reaction.

17. The process according to Claim 16, wherein the nonpolar solvent is benzene, toluene, xylene (o, m or p), ethylbenzene or 1,2,4-trimethylbenzene.

18. A catalyst used for hydrogenation reaction, comprising a rhodium compound and a metal compound.

19. The catalyst according to Claim 18, which further comprises an amine.

20. The catalyst according to Claim 18 or Claim 19, wherein the rhodium compound is rhodium-carbon, rhodium-alumina, rhodium-calcium carbonate or rhodium-barium sulfate.

21. The catalyst according to Claim 18 or Claim 19, wherein the metal compound is a nickel(II) compound, an iron(II) compound, an iron(III) compound, a cobalt(II) compound or a cobalt(III) compound.

22. The catalyst according to Claim 19, wherein the amine is a secondary amine or a tertiary amine.

23. The catalyst according to Claim 19, wherein the amine is pyrrolidine, piperidine, dimethylamine, diethylamine, diisopropylamine, dibutylamine, trimethylamine, triethylamine or tributylamine.

24. The catalyst according to Claim 21, wherein the nickel(II) compound, the iron(II) compound, the iron(III) compound, the cobalt(II) compound or the cobalt(III) compound are NiBr₂, Ni(NO₃)₂, Ni(OCOCH₃)₂, FeBr₃, FeCl₂, FeSO₄ , FeCl₃, FeCl₃-SiO₂, Fe(OCOCH₃)₂, Fe(II)fumarate, CoBr₂, CoCl₂, 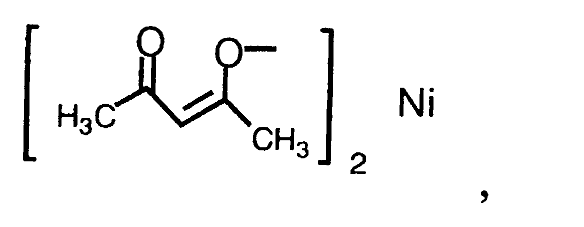 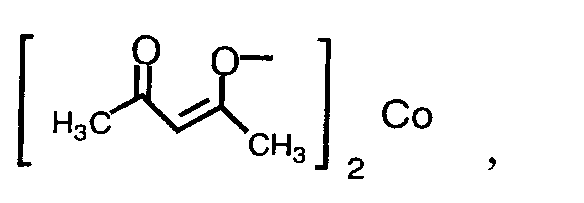 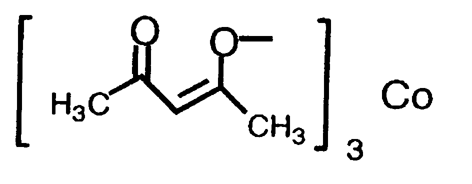 or
